# EUROPÄISCHE PATENTSCHRIFT

(11) **EP 2 791 331 B1**
(45) Veröffentlichungstag und Bekanntmachung des Hinweises auf die Patenterteilung: **15.03.2017**
(21) Anmeldenummer: 12805512.6
(22) Anmeldetag: 10.12.2012
(51) Int. Cl.: C12N 9/54, C11D 3/386

(54) **LAGERSTABILES FLÜSSIGES WASCH- ODER REINIGUNGSMITTEL ENTHALTEND PROTEASE UND AMYLASE**
STORAGE-STABLE LIQUID DETERGENT OR CLEANING AGENT CONTAINING PROTEASE AND AMYLASE
DÉTERGENT OU NETTOYANT LIQUIDE STABLE AU STOCKAGE CONTENANT UNE PROTÉASE ET UNE AMYLASE

(30) Priorität: 15.12.2011 DE 102011088751
(43) Veröffentlichungstag der Anmeldung: 22.10.2014
(73) Patentinhaber: Henkel AG & Co. KGaA, 40589 Düsseldorf (DE)
(72) Erfinder: MUSSMANN, Nina, 47877 Willich (DE); EITING, Thomas, 40589 Düsseldorf (DE); BASTIGKEIT, Thorsten, 42279 Wuppertal (DE); BENDA, Konstantin, 40217 Düsseldorf (DE); HELLMUTH, Hendrik, 40237 Düsseldorf (DE)
(86) Internationale Anmeldenummer: PCT/EP2012/074883
(87) Internationale Veröffentlichungsnummer: WO 2013/087545

(56) Entgegenhaltungen:
- WO-A2-2006/066596
- DE-A1-102009 029 513

### SEQUENCE LISTING

<110> Henkel AG & Co. KGaA
<120> Lagerstabiles flüssiges Wasch- oder Reinigungsmittel enthaltend Protease und Amylase
<130> PT019382 PCT
<150> DE 102011088751.2
   <151> 2011-12-15
<160> 4
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 2
   <211> 269
   <212> PRT
   <213> Bacillus lentus
<400> 2
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Glu Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 3
   <211> 269
   <212> PRT
   <213> Bacillus lentus
<400> 3
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Asp Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 4
   <211> 485
   <212> PRT
   <213> Bacillus sp.
<400> 4
   His His Asn Gly Thr Asn Gly Thr Met Met Gln Tyr Phe Glu Trp Tyr
   1 5 10 15
   Leu Pro Asn Asp Gly Asn His Trp Asn Arg Leu Arg Ser Asp Ala Ser
   20 25 30
   Asn Leu Lys Asp Lys Gly Ile Ser Ala Val Trp Ile Pro Pro Ala Trp
   35 40 45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Ile Arg Thr Lys Tyr Gly
   65                  70                  75                  80
   Thr Arg Asn Gln Leu Gln Ala Ala Val Asn Ala Leu Lys Ser Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Ser Gly Glu Tyr Thr Ile Glu Ala Trp Thr Lys Phe Asp
       130                 135                 140
   Phe Pro Gly Arg Gly Asn Thr His Ser Asn Phe Lys Trp Arg Trp Tyr
   145                 150                 155                 160
   His Phe Asp Gly Val Asp Trp Asp Gln Ser Arg Lys Leu Asn Asn Arg
                   165                 170                 175
   Ile Tyr Lys Phe Arg Gly Asp Gly Lys Gly Trp Asp Trp Glu Val Asp
               180                 185                 190
   Thr Glu Asn Gly Asn Tyr Asp Tyr Leu Met Tyr Ala Asp Ile Asp Met
           195                 200                 205
   Asp His Pro Glu Val Val Asn Glu Leu Arg Asn Trp Gly Val Trp Tyr
       210                 215                 220
   Thr Asn Thr Leu Gly Leu Asp Gly Phe Arg Ile Asp Ala Val Lys His
   225                 230                 235                 240
   Ile Lys Tyr Ser Phe Thr Arg Asp Trp Ile Asn His Val Arg Ser Ala
                   245                 250                 255
   Thr Gly Lys Asn Met Phe Ala Val Ala Glu Phe Trp Lys Asn Asp Leu
               260                 265                 270
   Gly Ala Ile Glu Asn Tyr Leu Asn Lys Thr Asn Trp Asn His Ser Val
           275                 280                 285
   Phe Asp Val Pro Leu His Tyr Asn Leu Tyr Asn Ala Ser Lys Ser Gly
       290                 295                 300
   Gly Asn Tyr Asp Met Arg Gln Ile Phe Asn Gly Thr Val Val Gln Arg
   305 310 315 320
   His Pro Met His Ala Val Thr Phe Val Asp Asn His Asp Ser Gln Pro
   325 330 335
   Glu Glu Ala Leu Glu Ser Phe Val Glu Glu Trp Phe Lys Pro Leu Ala
   340 345 350
   Tyr Ala Leu Thr Leu Thr Arg Glu Gln Gly Tyr Pro Ser Val Phe Tyr
   355 360 365
   Gly Asp Tyr Tyr Gly Ile Pro Thr His Gly Val Pro Ala Met Lys Ser
   370 375 380
   Lys Ile Asp Pro Ile Leu Glu Ala Arg Gln Lys Tyr Ala Tyr Gly Arg
   385 390 395 400
   Gln Asn Asp Tyr Leu Asp His His Asn Ile Ile Gly Trp Thr Arg Glu
   405 410 415
   Gly Asn Thr Ala His Pro Asn Ser Gly Leu Ala Thr Ile Met Ser Asp
   420 425 430
   Gly Ala Gly Gly Asn Lys Trp Met Phe Val Gly Arg Asn Lys Ala Gly
   435 440 445
   Gln Val Trp Thr Asp Ile Thr Gly Asn Arg Ala Gly Thr Val Thr Ile
   450 455 460
   Asn Ala Asp Gly Trp Gly Asn Phe Ser Val Asn Gly Gly Ser Val Ser
   465 470 475 480
   Ile Trp Val Asn Lys
   485

## Beschreibung

Die Erfindung liegt auf dem Gebiet der flüssigen Wasch- und Reinigungsmittel. Die Erfindung betrifft insbesondere flüssige enzymhaltige Wasch- und Reinigungsmittel, die definierte Proteasen in Kombination mit einer Amylase enthalten, und schlägt ferner Verfahren vor, in denen solche Mittel angewendet werden. Die Erfindung betrifft ferner Verwendungen definierter Proteasen in flüssigen Wasch- oder Reinigungsmitteln, die eine Amylase enthalten.

Für Wasch- und Reinigungsmittel werden bevorzugt Proteasen vom Subtilisin-Typ eingesetzt. Die in den aus dem Stand der Technik bekannten Wasch- oder Reinigungsmitteln eingesetzten Proteasen stammen entweder ursprünglich aus Mikroorganismen, beispielsweise der Gattungen Bacillus, Streptomyces, Humicola, Thermomyces oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Pilze.

Insbesondere in modernen Flüssigwaschmitteln sind zunehmend weitere Enzyme enthalten, hierunter insbesondere Amylasen. Eine Amylase ist ein Enzym, das die Hydrolyse von Glykosidbindungen katalysiert, insbesondere in Polysacchariden wie Stärke. Unter den Amylasen werden in Wasch- und Reinigungsmitteln oftmals α-Amylasen eingesetzt, die die α(1-4)-Glykosidbindungen der Amylose hydrolysieren. Innerhalb der EC-Klassifikation der Enzyme, dem numerischen Klassifikationssystem für Enzyme, weisen α-Amylasen die EC-Nummer (engl. "Enzyme Commission number") 3.2.1.1 auf und zählen folglich zur dritten der sechs Enzymhauptklassen, den Hydrolasen (E.C.3.-.-.-), hierunter zu den Glycosylasen (E.C. 3.2.-.-) und wiederum hierunter zu den Glycosidasen (E.C. 3.2.1.-), d.h. Enzymen, die O- und/oder S-Glycosyl-Verbindungen hydrolysieren. Beim Stärkeabbau durch α-Amylasen entstehen Dextrine und daraus Maltose, Glucose und verzweigte Oligosaccharide. Amylasen wirken folglich insbesondere gegen Stärke-haltige Rückstände in der Wäsche und katalysieren deren Hydrolyse.

In den internationalen Patentanmeldungen WO 95/23221 und WO 92/21760 sind Varianten der alkalischen Protease aus Bacillus lentus DSM 5483 offenbart, die für ihren Einsatz in Wasch- oder Reinigungsmitteln geeignet sind, sowie derartige Proteasen enthaltende Wasch- und Reinigungsmittel. Ferner sind in der internationalen Patentanmeldung WO 2011/032988 Wasch- und Reinigungsmittel offenbart, die ebenfalls Varianten der alkalischen Protease aus Bacillus lentus DSM 5483 enthalten. Die in diesen Schriften offenbarten Proteasevarianten können neben weiteren Positionen an den Positionen 3, 4, 99 und 199 in der Zählweise der alkalischen Protease aus Bacillus lentus DSM 5483 verändert sein und beispielsweise an den besagten Positionen die Aminosäuren 3T, 41, 99D, 99E oder 1991 aufweisen. Ferner ist offenbart, dass die Waschmittel weitere Enzyme enthalten können, hierunter auch eine Amylase. Die Waschmittel können fest oder flüssig sein. Jedoch geht aus dieser Schrift nicht unmittelbar und eindeutig ein flüssiges Waschmittel hervor, das eine Amylase in Kombination mit einer Protease enthält, die Kombinationen dieser Veränderungen, wie sie nachstehend beschrieben werden, aufweist.

Ein Nachteil von protease- und amylasehaltigen flüssigen Wasch- und Reinigungsmitteln aus dem Stand der Technik ist, dass sie nicht ausreichend lagerstabil sind und sie demnach bereits nach kurzer Zeit ein erhebliches Maß an enzymatischer, insbesondere an amylolytischer und/oder proteolytischer, Aktivität einbüßen. Häufig führt die Anwesenheit von Protease zum Verlust amylolytischer Aktivität, da die Protease die Amylase inaktiviert. Das Wasch- oder Reinigungsmittel zeigt dann keine optimale Reinigungsleistung mehr.

Der vorliegenden Erfindung liegt die Aufgabe zugrunde, den genannten Nachteil zu überwinden und protease- und amylasehaltige flüssige Wasch- oder Reinigungsmittel bereitzustellen, die ausreichend bzw. verbessert lagerstabil sind, insbesondere hinsichtlich ihrer enzymatischen und vorzugsweise ihrer amylolytischen und/oder proteolytischen Aktivität.

Ein Gegenstand der Erfindung ist daher ein flüssiges Wasch- oder Reinigungsmittel umfassend
(a) eine Protease, die eine Aminosäuresequenz umfasst, die zu der in SEQ ID NO. 1 angegebenen Aminosäuresequenz über deren Gesamtlänge zu mindestens 70% identisch ist und in der Zählung gemäß SEQ ID NO. 1 die Aminosäuresubstitution R99E oder R99D in Kombination mit den Aminosäuresubstitutionen S3T, V4I und V199I aufweist, und
(b) eine Amylase dadurch gekennzeichnet. dass die Amylase eine α-Amylase-Variante der α-Amylase AA560 gemäß SEQ ID NO. 4 ist, die
   (i) gegenüber der α-Amylase AA560 gemäß SEQ ID NO. 4 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K: oder
   (ii) gegenüber der α-Amylase AA560 gemäß SEQ ID NO_{.} 4 folgende Sequenzveränderungen in der Zählung der α-Amylase AA560 aufweist:
      - (1): M9L / M202I,
      - (2): M9L / M202I / M323T,
      - (3): M9L / M202I / M323T / M382Y,
      - (4): M9L / M202I / Y295F / A339S,
      - (5): M9L / M202I / Y295F,
      - (6): M9L / M202I / A339S,
      - (7): M9L / M202I / Y295F / A339S.
      - (8): M9L / M202I / Y295F / A339S / E345R,
      - (9): M9L / G149A / M202I / Y295F / A339S / E345R,
      - (10): M9L / M202L,
      - (11): M9L / M202L / M323T,
      - (12): M9L / M202L / M323T / M382Y.
      - (13): M9L / M202L / Y295F / A339S.
      - (14): M9L / M202L / Y295F,
      - (15): M9L / M202L 1 A339S,
      - (16): M9L / M202L / Y295F / A339S.
      - (17): M9L / M202L / Y295F / A339S, E345R.
      - (18): M9L / G149A / M202L / Y295F / A339S / E345R,
      - (19): M9L / M202T.
      - (20): M9L / M202T / M323T,
      - (21): M9L / M202T / M323T / M382Y,
      - (22): M9L / M202T / Y295F / A339S,
      - (23): M9L / M202T / Y295F,
      - (24): M9L / M202T / A339S,
      - (25): M9L / M202T / Y295F / A339S,
      - (26): M9L / M202T / Y295F / A339S / E345R,
      - (27): M9L / G149A / M202T / Y295F / A339S / E345R,
      - (28): M9L / G149A / M2021 / V214T / Y295F / N299Y / M323T / A339S / E345R;
      - (29): M9L / G149A / M202L / V214I / Y295F / M323T / A339S / E345R / M382Y,
      - (30): M9L / G149A / G182T / G186A / M2021 / V214I / Y295F / N299Y / M323T / A339S;
      - (31): M9L / G149A / G182T / G186A / M202L / T2571 / Y295F / N299Y / M323T / A339S / E345R,
      - (32): M9L / G149A / M202L / V214T / Y295F / N299Y / M323T / A339S / E345R,
      - (33): M9L / G149A / M202I / V214I / Y295F / M323T / A339S / E345R / M382Y,
      - (34): M9L / G149A / G182T / G186A / M202L / V2141 / Y295F / N299Y / M323T / A339S,
      - (35): M9L / G149A / G182T / G186A / M2021 / T2571 / Y295F / N299Y / M323T / A339S / E345R,
      - (36): M9L / G149A / M2021 / V214T / Y295F / N299Y / M323T / A339S / E345R / N471E,
      - (37): M9L / G149A / M202L / V2141 / Y295F / M323T / A339S / E345R / M382Y / N471E,
      - (38): M9L / G149A / G182T / G186A / M2021 / V2141 / Y295F / N299Y / M323T / A339S / N471E,
      - (39): M9L / G149A / G182T / G186A / M202L / T2571 / Y295F / N299Y / M323T / A339S / E345R / N471E,
      - (40): M202L / M105F / M208F,
      - (41): G133E / M202L / Q361E,
      - (42): G133E / M202L / R444E,
      - (43): M202L / Y295F,
      - (44): M202L / A339S,
      - (45): M202L / M323T,
      - (46): M202L / M323T / M309L,
      - (47): M202L / M323T / M430I,
      - (48): M202L / V214T / R444Y,
      - (49): M202L / N283D Q361E,
      - (50): M202L / M382Y / K383R,
      - (51): M202L / K446R / N484Q,
      - 52: M2021 / Y295F,
      - (53): M2021 / A339S,
      - (54): M2021 / M105F / M208F,
      - (55): G133E / M2021 / Q361 E,
      - (56): G133E / M2021 / R444E,
      - (57): M202I / M323T,
      - (58): M202I / M323T / M309L,
      - (59): M2021 / M323T / M4301,
      - (60): M2021 / V214T / R444Y,
      - (61): M2021 / N283D / Q361E,
      - (62): M2021 / M382Y / K383R,
      - (63): M2021 / K446R / N484Q,
      - (64): M202V / M105F / M208F,
      - (65): G133E / M202V / Q361E,
      - (66): G133E / M202V / R444E,
      - (67): M202V / M323T,
      - (68): M202V / M323T / M309L,
      - (69): M202V / M323T / M4301,
      - 70: M202V / M323T / M9L,
      - (71): M202V / V214T / R444Y,
      - 72: M202V / N283D Q361E,
      - (73): M202V / M382Y / K383R,
      - (74): M202V / K446R / N484Q,
      - (75): M202T / M105F / M208F,
      - (76): G133E / M202T / Q361E,
      - (77): G133E / M202T / R444E,
      - (78): M202T / Y295F,
      - (79): M202T / A339S,
      - (80): M202T / M323T,
      - (81): M202T / M323T / M309L,
      - (82): M202T / M323T / M4301,
      - (83): M202T / M323T / M9L,
      - (84): M202T / V214T / R444Y,
      - (85): M202T / N283D / Q361E,
      - (86): M202T / A339S,
      - (87): M202T / Y295F
      - (88): M202T / N299F,Y,
      - (89): M202T / M382Y / K383R oder
      - (90): M202T / K446R / N484Q

Überraschenderweise wurde festgestellt, dass ein flüssiges Wasch- oder Reinigungsmittel, welches die Kombination einer solchen Protease mit einer Amylase enthält, vorteilhaft lagerstabil ist. Insbesondere weist es eine verbesserte Reinigungsleistung, insbesondere eine verbesserte amylolytische und/oder proteolytische Reinigungsleistung, nach Lagerung auf im Vergleich mit einem Wasch- oder Reinigungsmittel, welches sich von einem erfindungsgemäßen Mittel lediglich durch die in dem jeweiligen Mittel vorhandene Protease unterschiedet, wobei in den zu vergleichenden Mitteln die Protease zu Lagerbeginn in gleicher Konzentration vorhanden ist, bezogen auf aktives Enzym. Eine im Rahmen der vorliegenden Erfindung vorgesehene Protease führt daher zu einer verminderten Inaktivierung der Amylase und zeigt auch selbst verminderte Leistungsverluste. Die verminderte Inaktivierung von Amylase und/oder Protease durch die im Rahmen der vorliegenden Erfindung vorgesehene Protease beruht jedoch nicht auf einer unzureichenden Proteaseleistung und/oder -aktivität.

Ein erfindungsgemäßes Mittel weist diesbezüglich und vorzugsweise eine weiterhin gute, insbesondere vorteilhafte, Reinigungsleistung an protease-sensitiven Anschmutzungen auf. Ein solches Mittel ermöglicht daher eine zufrieden stellende oder verbesserte Entfernung von mindestens einer, bevorzugt von mehreren protease-sensitiven Anschmutzungen auf Textilien und/oder harten Oberflächen, beispielsweise Geschirr. In ausgewählten Ausgestaltungen der Erfindung tritt eine solche Reinigungsleistung hinsichtlich mindestens einer protease-sensitiven Anschmutzung insbesondere auch bei niedrigen Temperaturen, auf, beispielsweise zwischen 10°C und 50°C, zwischen 10°C und 40°C oder zwischen 20°C und 40°C.

Hinsichtlich der einleitend erwähnten internationalen Patentanmeldungen WO 95/23221, WO 92/21760 und WO 2011/032988 handelt es bei der vorliegenden Erfindung somit um eine besonders vorteilhafte Auswahl, die zum Erhalt eines leistungsfähigen und lagerstabilen Flüssigwaschmittels führt, insbesondere hinsichtlich der proteolytischen und/oder amylolytischen Reinigungsleistung des Mittels nach Lagerung und/oder hinsichtlich der proteolytischen und/oder amylolytischen Aktivität des Mittels nach Lagerung.

Unter Reinigungsleistung wird im Rahmen der Erfindung das Vermögen des Wasch- oder Reinigungsmittels verstanden, eine vorhandene Anschmutzung teilweise oder vollständig bei der Anwendung des Mittels zu entfernen. Bei Wäscheanschmutzungen ist dies vorzugsweise die Aufhellungsleistung an einer oder mehreren Anschmutzungen auf Textilien. Beispiele für Wäscheanschmutzungen sind Blut-Milch/Tusche auf Baumwolle, Voll-Ei/Pigment auf Baumwolle, Schokolade-Milch/Tusche auf Baumwolle, Erdnuss Öl-Pigment/Tusche auf Polyester/Baumwolle, Gras auf Baumwolle oder Kakao auf Baumwolle. Bei Geschirrspülmitteln beschreibt die Reinigungsleistung das Vermögen des Geschirrspülmittels, eine vorhandene Anschmutzung von der harten Oberfläche des Geschirrs zu entfernen. Beispiele für Geschirranschmutzungen sind Milch, Hackfleisch, Eigelb, Haferflocken und Stärke. Im Rahmen der Erfindung weist sowohl das Wasch- oder Reinigungsmittel, welches die Protease und die Amylase umfasst bzw. die durch dieses Mittel gebildete Waschflotte, als auch die Protease bzw. die Amylase selbst eine jeweilige Reinigungsleistung auf. Die Reinigungsleistung der Enzyme trägt somit zur Reinigungsleistung des Mittels bzw. der durch das Mittel gebildeten Waschflotte bei. Die amylolytische Reinigungsleistung bezeichnet die Reinigungsleistung an amylase-sensitiven Anschmutzungen. Die proteolytische Reinigungsleistung bezeichnet die Reinigungsleistung an protease-sensitiven Anschmutzungen. Die Reinigungsleistung wird in fachüblicher Art und Weise ermittelt, vorzugsweise wie weiter unten angegeben.

Unter Waschflotte wird diejenige das Wasch-oder Reinigungsmittel enthaltende Gebrauchslösung verstanden, die auf Textilien oder Gewebe bzw. harte Oberflächen einwirkt und damit mit den auf Textilien bzw. Geweben oder harten Oberflächen vorhandenen Anschmutzungen in Kontakt kommt. Üblicherweise entsteht die Waschflotte, wenn der Wasch- oder Reinigungsvorgang beginnt und das Wasch- oder Reinigungsmittel beispielsweise in einer Geschirrspülmaschine, einer Waschmaschine oder in einem anderen geeigneten Behältnis mit Wasser verdünnt wird.

Eine Lagerstabilität im Sinne der Erfindung liegt insbesondere dann vor, wenn ein erfindungsgemäßes Wasch- oder Reinigungsmittel nach einer Lagerung eine höhere Reinigungsleistung aufweist im Vergleich zu einer Kontrollzusammensetzung, die sich nur durch die in der Kontrollzusammensetzung enthaltene Protease von dem erfindungsgemäßen Wasch-oder Reinigungsmittel unterscheidet. Beide zu vergleichenden Mittel weisen bei Lagerbeginn daher die gleiche Amylasemenge bzw. -konzentration und/oder amylolytische Ausgangsaktivität auf. Weiterhin ist in beiden Mitteln die Protease zu Lagerbeginn in gleicher Konzentration vorhanden, bezogen auf aktives Enzym, und beide Mittel werden auf die gleiche Art und Weise behandelt, insbesondere betreffend die Bedingungen der Lagerung und die Bestimmung der Enzymaktivität. Zunehmend bevorzugt erfolgt die Lagerung für mindestens 24 Stunden, 48 Stunden, 72 Stunden, 5 Tage, 1 Woche, 2 Wochen, 3 Wochen oder 4 Wochen. Weiter bevorzugt erfolgt die Lagerung bei einer Temperatur von 20°C, 30°C oder 40°C, besonders bevorzugt bei 40°C.

Die Bestimmung der Enzymaktivität kann diesbezüglich - abgestimmt auf den jeweiligen Enzymtyp - in fachüblicher Art und Weise erfolgen. Methoden zur Aktivitätsbestimmung sind dem Fachmann auf dem Gebiet der Enzymtechnologie geläufig und werden von ihm routinemäßig angewendet. Verfahren zur Bestimmung der Proteaseaktivität sind beispielsweise offenbart in Tenside, Band 7 (1970), S. 125-132. Die proteolytische Aktivität kann ferner bestimmt werden über die Freisetzung des Chromophors para-Nitroanilin (pNA) aus dem Substrat suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6 und einer Wellenlänge von 410 nm. Die Messzeit beträgt 5 min. bei einem Messintervall von 20s bis 60s. Die Proteaseaktivität wird vorzugsweise in PE (Protease-Einheiten) angegeben.

Die Amylaseaktivität wird in fachüblicher Weise bestimmt. Vorzugsweise wird die Amylaseaktivität bestimmt wie nachstehend angegeben. Amylasen setzen Stärke zu Glucose um. Unter definierten Reaktionsbedingungen (Tris-maleatpuffer pH 6,5, 50°C, 15 min) werden die zu untersuchenden Proben mit 0,67% Stärke (löslich, vorbehandelt nach Zulkowsky (behandelt mit Glycerin bei 190°C)) inkubiert. Durch Zugabe von Dinitrosalicylsäure und Erhitzen auf 100°C wird diese durch Glukose und andere reduzierende Zucker unter alkalischen Bedingungen zu einem orangeroten Farbstoff reduziert, was nach Beendigung der Reaktion photometrisch bei 540 nm bestimmt wird. Die der Färbung entsprechende Menge des freigesetzten Zuckers ist dabei ein Maß für die Enzymaktivität (vgl. Sumner et al., J. Biol. Chem., 1921, 47 & 1924, 62).

Besonders bevorzugt wird das Vorliegen einer Enzymstabilisierung im Sinne der vorliegenden Erfindung ermittelt wie vorstehend angegeben unter Verwendung eines Protease- und Amylasehaltigen flüssigen Wasch- oder Reinigungsmittels, das für vier Wochen bei einer Temperatur von 40°C gelagert wird, wobei die proteolytische Aktivität bestimmt wird über die Freisetzung des Chromophors para-Nitroanilin (pNA) aus dem Substrat suc-AAPF-pNA, und die amylolytische Aktivität bestimmt wird wie vorstehend angegeben.

Die in einem erfindungsgemäßen Wasch- oder Reinigungsmittel enthaltene Protease umfasst eine Aminosäuresequenz, die zu der in SEQ ID NO. 1 angegebenen Aminosäuresequenz über deren Gesamtlänge zu mindestens 70% identisch ist und in der Zählung gemäß SEQ ID NO. 1 die Aminosäuresubstitution R99E oder R99D in Kombination mit den Aminosäuresubstitutionen S3T, V4I und V199I aufweist.

In einer weiteren Ausführungsform der Erfindung umfasst die Protease eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und in der Zählung gemäß SEQ ID NO. 1 die Aminosäuresubstitution R99E in Kombination mit den Aminosäuresubstitutionen S3T, V4I und V199I aufweist.

In einer weiteren Ausführungsform der Erfindung umfasst die Protease eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und in der Zählung gemäß SEQ ID NO. 1 die Aminosäuresubstitution R99D in Kombination mit den Aminosäuresubstitutionen S3T, V41 und V199I aufweist.

SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

Erfindungsgemäßer Proteasen zeichnen sich dadurch aus, dass sie die Aminosäuresubstitution R99E oder R99D in Kombination mit den drei weiteren Aminosäuresubstitutionen S3T, V4I und V199I aufweisen. Insbesondere folgende Proteasen sind diesbezüglich ganz besonders bevorzugt:
Protease umfassend eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5% 98% und 98,5% identisch ist, und in der Zählung gemäß SEQ ID NO. 1 die Aminosäuresubstitution R99E in Kombination mit den Aminosäuresubstitutionen S3T, V4I und V199I aufweist, insbesondere eine Protease gemäß SEQ ID NO. 1 mit den Aminosäuresubstitutionen S3T, V4I, R99E und V199I. Eine derartige Protease in SEQ ID NO. 2 angegeben.
Protease umfassend eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%. 83%, 84%, 85%. 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist, und in der Zählung gemäß SEQ ID NO. 1 die Aminosäuresubstitution R99D in Kombination mit den Aminosäuresubstitutionen S3T, V4I und V199I aufweist, insbesondere eine Protease gemäß SEQ ID NO. 1 mit den Aminosäuresubstitutionen S3T, V4I, R99D und V199I. Eine derartige Protease ist in SEQ ID NO. 3 angegeben.

Weitere besonders bevorzugte Proteasen sind Proteasen wie vorstehend beschrieben, die ferner an Position 211 in der Zählung gemäß SEQ ID NO. 1 die Aminosäure Leucin (L) aufweisen.

Die Aminosäurepositionen werden im Rahmen der vorliegenden Erfindung durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1. Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind:
Neben Position 99 besonders vorteilhafte Positionen sind demnach die Positionen 3, 4, 199 und 211, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, V199, und L211. In Abhängigkeit von der Anzahl der vorliegenden Sequenzabweichungen von SEQ ID NO.1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der erfindungsgemäß vorgeschlagenen Sequenzveränderungen im Einzelfall zu berücksichtigen und ist ferner auch abhängig von der Länge der Aminosäuresequenz der Protease. Beispielsweise beträgt die maximale Identität bei drei, vier, fünf, sechs, sieben, acht oder neun Sequenzveränderungen 98,88%, 98,51%, 98,14%, 97,77%, 97,40%, 97,03% bzw. 96,65% bei einer 269 Aminosäuren langen Aminosäuresequenz, beziehungsweise 98,91%, 98,55%, 98,18%, 97,82%, 97,45%, 97,09% bzw. 96,73% bei einer 275 Aminosäuren langen Aminosäuresequenz.

Erfindungsgemäß hat sich gezeigt, dass durch den Zusatz einer solchen Protease zu einem flüssigen Wasch- oder Reinigungsmittel, welches eine Amylase enthält, ein besonders lagerstabiles Flüssigwaschmittel erhalten wird, insbesondere hinsichtlich dessen verbleibender Reinigungsleistung nach Lagerung, insbesondere nach einer Lagerdauer von zunehmend bevorzugt.24 Stunden, 48 Stunden, 72 Stunden, 5 Tage, 1 Woche, 2 Wochen, 3 Wochen oder 4 Wochen.

Eine in einem erfindungsgemäßen Wasch- oder Reinigungsmittel enthaltene Protease weist eine proteolytische Aktivität auf, das heißt, sie ist zur Hydrolyse von Peptidbindungen eines Polypeptids bzw. Proteins befähigt. Sie ist daher ein Enzym, welches die Hydrolyse von Peptidbindungen katalysiert und dadurch in der Lage ist, Peptide oder Proteine zu spalten. Sie ist insbesondere eine Subtilase und besonders bevorzugt ein Subtilisin.

Eine Amylase ist ein Enzym wie einleitend beschrieben. Für Amylasen können synonyme Begriffe verwendet werden, beispielsweise 1,4-alpha-D-Glucan-Glucanohydrolase oder Glycogenase. Erfindungsgemäß konfektionierbare Amylasen sind vorzugsweise α-Amylasen. Entscheidend dafür, ob ein Enzym eine α-Amylase im Sinne der Erfindung ist, ist deren Fähigkeit zur Hydrolyse von α(1-4)-Glykosidbindungen in der Amylose der Stärke.

Erfindungsgemäß konfektionierbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purasta®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Desweiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme. ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Besonders geeignet für den Einsatz in erfindungsgemäßen Mitteln sind folgende α-Amylase-Varianten:
(a) α-Amylase-Varianten, die gegenüber der α-Amylase AA560 gemäß SEQ ID NO 4 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K. Besonders bevorzugt weist die α-Amylase-Variante alle sechs der genannten Sequenzveränderungen auf.
(b) α-Amylase-Varianten, die gegenüber der α-Amylase AA560 gemäß SEQ ID NO. 4 folgende Sequenzveränderungen aufweist (in der Zählung der α-Amylase AA560):
   - (1): M9L / M202I,
   - (2): M9L / M202I / M323T,
   - (3): M9L / M202I / M323T / M382Y,
   - (4): M9L / M202I / Y295F / A339S,
   - (5): M9L / M202I / Y295F,
   - (6): M9L / M202I / A339S,
   - (7): M9L / M202I / Y295F / A339S,
   - (8): M9L / M202I / Y295F / A339S / E345R,
   - (9): M9L / G149A / M202I / Y295F / A339S / E345R,
   - (10): M9L / M202L,
   - (11): M9L / M202L / M323T,
   - (12): M9L / M202L / M323T / M382Y,
   - (13): M9L / M202L / Y295F / A339S,
   - (14): M9L / M202L / Y295F,
   - (15): M9L / M202L / A339S,
   - (16): M9L / M202L / Y295F / A339S,
   - (17): M9L / M202L / Y295F / A339S, E345R,
   - (18): M9L / G149A / M202L / Y295F / A339S / E345R,
   - (19): M9L / M202T,
   - (20): M9L / M202T / M323T,
   - (21): M9L / M202T / M323T / M382Y,
   - (22): M9L / M202T / Y295F / A339S,
   - (23): M9L / M202T / Y295F,
   - (24): M9L / M202T / A339S,
   - (25): M9L / M202T / Y295F / A339S,
   - (26): M9L / M202T / Y295F / A339S / E345R,
   - (27): M9L / G149A / M202T / Y295F / A339S / E345R,
   - (28): M9L / G149A / M202I / V214T / Y295F / N299Y / M323T / A339S / E345R,
   - (29): M9L / G149A / M202L / V214I / Y295F / M323T / A339S / E345R / M382Y,
   - (30): M9L / G149A / G182T / G186A / M2021 / V214I / Y295F / N299Y / M323T / A339S,
   - (31): M9L / G149A / G182T / G186A / M202L / T257I / Y295F / N299Y / M323T / A339S / E345R,
   - (32): M9L / G149A / M202L / V214T / Y295F / N299Y / M323T / A339S / E345R,
   - (33): M9L / G149A / M202I / V214I / Y295F / M323T / A339S / E345R / M382Y,
   - (34): M9L / G149A / G182T / G186A / M202L / V214I / Y295F / N299Y / M323T / A339S,
   - (35): M9L / G149A / G182T / G186A / M202I / T257I / Y295F / N299Y / M323T / A339S / E345R,
   - (36): M9L / G149A / M202I / V214T / Y295F / N299Y / M323T / A339S / E345R / N471E,
   - (37): M9L / G149A / M202L / V214I / Y295F / M323T / A339S / E345R / M382Y / N471E,
   - (38): M9L / G149A / G182T / G186A / M202I / V214I / Y295F / N299Y / M323T / A339S / N471E,
   - (39): M9L / G149A / G182T / G186A / M202L / T257I / Y295F / N299Y / M323T / A339S / E345R / N471E,
   - (40): M202L / M105F / M208F,
   - (41): G133E / M202L / Q361E, (42) G133E / M202L / R444E,
   - (43): M202L / Y295F,
   - (44): M202L / A339S,
   - (45): M202L / M323T,
   - (46): M202L / M323T / M309L,
   - (47): M202L / M323T / M430I,
   - (48): M202L / V214T / R444Y,
   - (49): M202L / N283D / Q361 E,
   - (50): M202L / M382Y / K383R,
   - (51): M202L / K446R / N484Q,
   - (52): M202I / Y295F,
   - (53): M202I / A339S,
   - (54): M202I / M105F / M208F,
   - (55): G133E / M202I / Q361E,
   - (56): G133E / M202I R444E,
   - (57): M202I / M323T,
   - (58): M202I / M323T / M309L,
   - (59): M202I / M323T / M430I,
   - (60): M202I / V214T / R444Y,
   - (61): M202I / N283D / Q361E,
   - (62): M202I / M382Y / K383R,
   - (63): M202I / K446R / N484Q,
   - (64): M202V / M105F / M208F,
   - (65): G133E / M202V / Q361E,
   - (66): G133E / M202V / R444E,
   - (67): M202V / M323T,
   - (68): M202V / M323T / M309L,
   - (69): M202V / M323T / M430I,
   - (70): M202V / M323T / M9L,
   - (71): M202V / V214T / R444Y,
   - (72): M202V / N283D / Q361E,
   - (73): M202V / M382Y / K383R,
   - (74): M202V / K446R / N484Q,
   - (75): M202T / M105F / M208F,
   - (76): G133E / M202T / Q361 E,
   - (77): G133E / M202T / R444E,
   - (78): M202T / Y295F,
   - (79): M202T / A339S,
   - (80): M202T / M323T,
   - (81): M202T / M323T / M309L,
   - (82): M202T / M323T / M430I,
   - (83): M202T / M323T / M9L,
   - (84): M202T / V214T / R444Y,
   - (85): M202T / N283D / Q361E,
   - (86): M202T / A339S,
   - (87): M202T / Y295F
   - (88): M202T / N299F,Y,
   - (89): M202T / M382Y / K383R oder
   - (90): M202T / K446R / N484Q

   Hierunter ganz besonders bevorzugt sind folgende α-Amylase-Varianten:
   - (10): M9L / M202L,
   - (28): M9L / G149A / M202I / V214T / Y295F / N299Y / M323T / A339S / E345R,
   - (31): M9L / G149A / G182T / G186A / M202L / T257I / Y295F / N299Y / M323T / A339S / E345R,
   - (35): M9L / G149A / G182T / G186A / M202I / T257I / Y295F / N299Y / M323T /
   - (38): M9L / G149A / G182T / G186A / M202I / V214I / Y295F / N299Y / M323T /
   - (39): M9L / G149A / G182T / G186A / M202L / T257I / Y295F / N299Y / M323T / A339S / E345R / N471 E,
   - (45): M202L / M323T,
   - (46): M202L / M323T / M309L,
   - (62): M202I / M382Y / K383R,
   - (68): M202V / M323T / M309L,
   - (73): M202V / M382Y / K383R
   - (82): M202T / M323T / M430I oder
   - (84): M202T / V214T / R444Y
(c) α-Amylase-Variante gemäß (b), die zusätzlich alle sechs unter (a) genannten Sequenzveränderungen aufweist, hierunter ganz besonders bevorzugt Variante 31 mit den sechs unter (a) genannten Sequenzveränderungen.

Erfindungsgemäß ganz besonders bevorzugt ist die vorstehend unter (a) genannte α-Amylase-Variante sowie die unter (c) genannte α-Amylase-Variante 31 mit den sechs unter (a) genannten Sequenzveränderungen.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl: beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

In einer weiteren Ausführungsform der Erfindung ist ein erfindungsgemäßes Wasch- oder Reinigungsmittel ferner dadurch gekennzeichnet, dass seine Reinigungsleistung mindestens derjenigen eines Wasch- bzw. Reinigungsmittels entspricht, das eine Protease beinhaltet, die eine Aminosäuresequenz umfasst, die der in SEQ ID NO. 2 oder SEQ ID NO. 3, besonders bevorzugt der in SEQ ID NO. 2, angegebenen Aminosäuresequenz entspricht. Die Reinigungsleistung wird in einem Waschsystem bestimmt, das ein amylasehaltiges Waschmittel in einer Dosierung zwischen 4,0 und 11,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Hackfleisch- und/oder einer Eigelb-Anschmutzung auf Geschirr ermittelt wird durch Bestimmung des verbleibenden Rückstands der jeweiligen Anschmutzung nach dem Waschvorgang, der Waschvorgang für mindestens 30 Minuten, vorzugsweise 60 Minuten, bei einer Temperatur von 50°C erfolgt und das Wasser eine Wasserhärte zwischen 15,5 und 16,5° dH (deutsche Härte) aufweist.

Bei dem Waschmittel für das Waschsystem handelt es sich vorzugsweise um ein zweiphasiges flüssiges maschinelles Geschirrspülmittel, das wie folgt zusammengesetzt ist (alle Angaben in Gewichts-Prozent):

### (a) Enzymphase:

| | |
|---|---|
| Builder | 15,0-20,0 |
| Zuckeralkohol | 8,0-12,0 |
| Nichtionisches Tensid (C8-C10 Fettalkoholethoxylat mit 22 EO) | 3,0-5,0 |
| Alkaliverbindung (Base) | 3,0-4,0 |
| Borsäure | 2,5-3,5 |
| Phosphonat (HEDP) | 1,5-2,5 |
| Amylase | 1,0-2,0 |
| Protease | siehe Text |
| Ca-Salz | 0,8-1,2 |
| Zn-Salz | 0,15-0,25 |
| Verdicker | 0,8-1,2 |
| Farbstoff, Parfüm, Konservierungsmittel | 0,25-0,5 |
| Wasser | ad 100 |

Bei der Amylase handelt es sich vorzugsweise um die Präparation einer α-Amylase-Variante, die gegenüber der α-Amylase AA560 gemäß SEQ ID NO. 4 folgende Sequenzveränderungen in der Zählung der α-Amylase AA560 aufweist: R118K, D183* (Deletion), G184* (Deletion), N195F, R320K, R458K (Firma Novozymes).

### (b) Alkalische Phase:

| | |
|---|---|
| Builder | 7,5-12,5 |
| Natriumcarbonat | 7,5-12,5 |
| Sulfopolymer | 5,0-8,0 |
| Alkaliverbindung (Base) | 3,0-5,0 |
| Monoethanolamin | 2,0-4,0 |
| Phosphonat (HEDP) | 2,0-5,0 |
| Verdicker | 0,8-1,2 |
| Farbstoff, Parfüm, Konservierungsmittel | 0,25-0,5 |
| Wasser | ad 100 |

Die Protease ist in dem Mittel in einer Konzentration von 0,01-1 Gew.-%, vorzugsweise von 0,1 bis 0,5 Gew.-%, vorhanden, bezogen auf aktives Protein. Für einen Waschgang in der Geschirrspülmaschine werden beide Phasen zu gleichen Teilen dosiert (jeweils 20g pro Phase). Gewaschen wird in einem pH-Wertebereich zwischen pH 9 und pH 10 in einer üblichen Geschirrspülmaschine, beispielsweise einer Geschirrspülmaschine G698SC der Firma Miele. Weder die Protease- noch die Amylaseaktivität in der Waschflotte sind zu Waschbeginn gleich Null.

Die Auswertung der Reinigungsleistung erfolgt gemäß der Standard IKW-Methode visuell anhand einer Skala von 1 bis 10, wobei der Wert 10 die beste Note ist (kein erkennbarer Rückstand).

Besonders bevorzugt erfolgt die Ermittlung der Reinigungsleistung in einer Geschirrspülmaschine gegenüber einer Hackfleisch- und einer Eigelb-Anschmutzung auf Geschirr unter Verwendung eines zweiphasigen flüssigen maschinellen Geschirrspülmittels wie vorstehend beschrieben.

In einer weiteren Ausführungsform der Erfindung ist ein erfindungsgemäßes Wasch- oder Reinigungsmittel ferner dadurch gekennzeichnet, dass seine Lagerstabilität mindestens derjenigen eines Wasch- bzw. Reinigungsmittels entspricht, das eine Protease beinhaltet, die eine Aminosäuresequenz umfasst, die der in SEQ ID NO. 2 oder SEQ ID NO. 3, besonders bevorzugt der in SEQ ID NO. 2, angegebenen Aminosäuresequenz entspricht. Eine solche Lagerstabilität liegt dann vor, wenn das erfindungsgemäße Wasch- oder Reinigungsmittel nach einer Lagerung von vier Wochen bei 50°C eine gleiche oder höhere Reinigungsleistung aufweist als das zum Vergleich heranzuziehende Wasch- oder Reinigungsmittel, wobei sich das erfindungsgemäße Mittel nur durch die enthaltene Protease von dem als Vergleich heranzuziehenden Wasch- oder Reinigungsmittel unterscheidet.

Besonders bevorzugt handelt es sich bei dem zum Vergleich heranzuziehenden Mittel um ein zweiphasiges flüssiges maschinelles Geschirrspülmittel wie vorstehend angegeben, wobei die Reinigungsleistung bestimmt wird wie vorstehend angegeben.

Bei Lagerbeginn weisen beide zu vergleichenden Mittel die gleiche amylolytische Ausgangsaktivität auf, enthalten die Protease in gleicher Konzentration bezogen auf aktives Enzym, und beide Mittel werden auf die gleiche Art und Weise behandelt. Die proteolytische Aktivität in den Mitteln wird jeweils bestimmt über die Freisetzung des Chromophors para-Nitroanilin (pNA) aus dem Substrat suc-AAPF-pNA, und deren amylolytische Aktivität wird jeweils bestimmt wie vorstehend angegeben. Die Ausgangsaktivitäten für die Protease und die Amylase in dem jeweiligen Mittel sind nicht gleich Null.

Durch den jeweils aktivitätsgleichen Einsatz der Amylase und den konzentrationsgleichen Einsatz der Proteasen, bezogen auf aktives Protein, wird sichergestellt, dass auch bei einem etwaigen Auseinanderklaffen des Verhältnisses von Aktivsubstanz zu Gesamtprotein (die Werte der spezifischen Aktivität) die real vorliegenden enzymatischen Eigenschaften verglichen werden.

Soweit nicht anders angegeben wird im Rahmen der vorliegenden Erfindung jeweils auf das Gewicht des flüssigen Waschmittels Bezug genommen, d.h. die Angaben sind bezogen auf dessen Gewicht.

Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen. Bedingungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

Ferner kann auch die reife Protease an ihrem N-terminalen und/oder C-terminalen Ende verkürzt sein, so dass eine gegenüber SEQ ID NO. 1 bzw. SEQ ID NO. 2 oder SEQ ID NO. 3 verkürzte Protease, also ein Fragment, in dem erfindungsgemäßen Wasch- oder Reinigungsmittel enthalten ist. Alle Identitätsangaben beziehen sich in diesem Fall auf denjenigen Bereich, in dem das jeweilige Fragment in einem Alignment SEQ ID NO. 1 zugeordnet ist. Das jeweilige Fragment beinhaltet aber in jedem Fall diejenigen Positionen, die den Positionen 3, 4, 99 und/oder 199 in einem Alignment mit SEQ ID NO. 1 zugeordnet sind, und weist hier entsprechende Veränderungen wie erfindungsgemäß vorgesehen auf. Ferner ist ein solches Fragment proteolytisch aktiv. Ein diesbezüglich weiter bevorzugtes Fragment umfasst eine Aminosäuresequenz, die über eine Länge von mindestens 100 oder mindestens 110, 120, 130, 140, 150, 160, 170, 180, 190, 200, 210, 220, 230, 240, 250, 260, 265 oder 266 zusammenhängenden Aminosäurepositionen mit SEQ ID NO. 1 oder SEQ ID NO. 2 oder SEQ ID NO. 3 übereinstimmt unter Berücksichtigung der vorstehend genannten Aminosäuren für die Position 99 und ferner für die Positionen 3 und/oder 4 und/oder 199 und/oder 211. Besonders bevorzugt entspricht die Reinigungsleistung und/oder Lagerstabilität eines erfindungsgemäßen flüssigen Wasch- oder Reinigungsmittels mit einem solchen Fragment mindestens derjenigen eines Wasch- bzw. Reinigungsmittels, das eine Protease beinhaltet, die eine Aminosäuresequenz umfasst, die der in SEQ ID NO. 2 oder SEQ ID NO. 3 angegebenen Aminosäuresequenz entspricht, jeweils bestimmt wie vorstehend angegeben.

Ein erfindungsgemäßes Mittel enthält die Protease zunehmend bevorzugt in einer Menge von 1 x 10⁻⁸-5 Gew.-%, von 0,0001-3 Gew.-%, von 0,0005-1 Gew.-%, von 0,001 bis 0,75 Gew.-% und besonders bevorzugt von 0,005 bis 0,5 Gew.-%, bezogen auf aktives Protein. Ein erfindungsgemäßes Mittel enthält die Amylase zunehmend bevorzugt in einer Menge von 1 x 10⁻⁸-5 Gew.-%, von 0,0001-3 Gew.-%, von 0,0005-1 Gew.-%, von 0,001 bis 0,75 Gew.-% und besonders bevorzugt von 0,005 bis 0,5 Gew.%, bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Protease und/oder die Amylase können ferner an Trägerstoffen adsorbiert und/oder in Hüll-substanzen eingebettet sein, um sie gegen vorzeitige Inaktivierung zu schützen. In der Waschflotte, also unter Anwendungsbedingungen, wird das Enzym dann freigesetzt und kann seine katalytische Wirkung entfalten.

In einer weiteren Ausführungsform der Erfindung ist das Wasch- oder Reinigungsmittel dadurch gekennzeichnet, dass es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

Es wurde festgestellt, dass der Zusatz solcher Substanzen die Reinigungsleistung von Wasch- und Reinigungsmitteln, insbesondere von flüssigen Wasch- oder Reinigungsmitteln, welche Proteasen und Amylasen enthalten, insbesondere solche wie vorstehend beschrieben, weiter verbessert, insbesondere bei vergleichsweise niedrigen Temperaturen, insbesondere zwischen 10°C und 50°C, zwischen 10°C und 40°C, zwischen 10°C und 30°C und/oder zwischen 20°C und 40°C. Insbesondere in Kombination mit einer erfindungsgemäß einzusetzenden Protease tritt eine synergistische Wirkung auf, vor allem hinsichtlich der Entfernung von mindestens einer protease-sensitiven Anschmutzung, insbesondere einer solchen wie vorstehend angegeben.

Bei den vorstehend unter i. angegebenen Substanzen handelt es sich um anionische oder polyanionische Substanzen, d.h. diese Substanzen tragen mindestens eine und bevorzugt mehrere negative Ladungen. Bevorzugt handelt es sich um ein Polymer mit mindestens einem negativ geladenen Monomer, bevorzugt mit mehreren negativ geladenen Monomeren. Erfindungsgemäß bevorzugt ist dieses Polymer daher ein negativ geladenes Polymer. Bevorzugt sind beispielsweise Polymere organischer Säuren bzw. deren Salze, insbesondere Polyacrylate und/oder Poly-Zuckersäuren und/oder Polyarcylat-copolymere und/oder Poly-zucker-copolymere. Diesbezüglich weitere bevorzugte Verbindungen sind Polyacrylsulfonate oder Polycarboxylate und deren Salze, Copolymere oder Salze der Coploymere.

Beispiele für besonders bevorzugt einzusetzende Substanzen sind Acusol 587D (Polyacrylsulfonat; Unternehmen Rohm & Haas/Dow Chemical), Acusol 445N (Polycarboxylat Natriumsalz; Unternehmen Rohm & Haas/Dow Chemical), Acusol 590 (Polyarcrylat-copolymer; Unternehmen Rohm & Haas/Dow Chemical), Acusol 916 (Polyarcrylat Natriumsalz; Unternehmen Rohm & Haas/Dow Chemical), Sokalan CP42 (modifiziertes Polycarboxylat Natriumsalz; Unternehmen BASF), Sokalan PA 30CL (Polycarboxylat Natriumsalz; Unternehmen BASF), Dequest P 9000 (Polymaleinsäure; Unternehmen Thermphos), Alginsäure, Poly-2-acrylamido-2-methyl-1-propan-sulfonsäure, Poly-4-styrol sulfonsäure -co-maleinsäure Natriumsalz, Poly-acrylamido-co-acrylsäure Natriumsalz, Poly-methacrylsäure Natriumsalz, Poly-methyl vinyl ether-alt maleinsäure oder Polyvinylsulfonsäure Natriumsalz.

Bei den unter ii. angegebenen Substanzen handelt es sich kationische oder polykationische Substanzen, d.h. diese Substanzen tragen mindestens eine und bevorzugt mehrere positive Ladungen. Bevorzugt handelt es sich um ein Polymer mit mindestens einem positiv geladenen Monomer, bevorzugt mit mehreren positiv geladenen Monomeren. Erfindungsgemäß bevorzugt ist dieses Polymer daher ein positiv geladenes Polymer. Beispiele für diesbezüglich bevorzugte Verbindungen sind Salze der Polyamine, Polyethyleneimine bzw. deren Copolymere, Salze der Polyallylamine, Salze der Polydiallyldimethylammonium-verbindungen oder Poly(acrylamide-co-diallyldimethylammonium-verbindungen.

Bei den unter iii. angegebenen Substanzen handelt es sich um Substanzen, die mindestens eine Hydroxyl- und/oder Polyhydroxyl-Gruppe und bevorzugt mehrere Hydroxyl- und/oder Polyhydroxyl-Gruppen aufweisen. Bevorzugt sind diesbezüglich beispielsweise Polyvinylalkohole, beispielsweise solche, die unter dem Handelsnamen Mowiol verfügbar sind (Unternehmen Kremer Pigmente GmbH & Co. KG).

Es wird an dieser Stelle ausdrücklich darauf hingewiesen, dass eine konkrete Substanz zu einer oder mehreren der vorstehend genannten Gruppen i. bis iii. zugehörig sein kann. Beispielsweise kann es sich um ein anionisches Polymer handeln, welches eine oder mehrere Hydroxyl- und/oder Polyhydroxyl-Gruppe(n) aufweist. Eine solche Substanz ist dann zugehörig zu den Gruppen i. und iii. Ebenso ist ein kationisches Polymer, welches eine oder mehrere Hydroxyl- und/oder Polyhydroxyl-Gruppe(n) aufweist, zugehörig zu den Gruppen ii. und iii.

Im Rahmen der vorliegenden Erfindung ebenfalls einsetzbar sind Derivate der vorstehend als zugehörig zu i., ii. oder iii. genannten Substanzen. Unter einem Derivat wird im Sinne der vorliegenden Anmeldung eine solche Substanz verstanden, die ausgehend von einer der vorstehend genannten Substanzen chemisch modifiziert ist, beispielsweise durch die Umwandlung einer Seitenkette oder durch kovalente Bindung einer anderen Verbindung an die Substanz. Bei solch einer Verbindung kann es sich beispielsweise um niedrigmolekulare Verbindungen wie Lipide oder Mono-, Oligo- oder Polysaccharide oder Amine bzw. Aminverbindungen handeln. Ferner kann die Substanz glykosyliert, hydrolysiert, oxidiert, N-methyliert, N-formyliert, N-acetyliert sein oder Methyl, Formyl, Ethyl, Acetyl, t-Butyl, Anisyl, Benzyl, Trifluroacetyl, N-hydroxysuccinimide, t-Butyloxycarbonyl, Benzoyl, 4-Methylbenzyl, Thioanizyl, Thiocresyl, Benzyloxymethyl, 4-Nitrophenyl, Benzyloxycarbonyl, 2-Nitrobenzoyl, 2-Nitrophenylsulphenyl, 4-Toluenesulphonyl, Pentafluorophenyl, Diphenylmethyl, 2-Chlorobenzyloxycarbonyl, 2,4,5-trichlorophenyl, 2-bromobenzyloxycarbonyl, 9-Fluorenylmethyloxycarbonyl, Tripheylmethyl, 2,2,5,7,8-pentamethyl-chroman-6-sulphonyl enthalten. Ebenso ist unter einem Derivat die kovalente oder nichtkovalente Bindung der Substanz an einen makromolekularen Träger zu verstehen, genauso wie auch ein nichtkovalenter Einschluss in geeignete makromolekulare Käfigstrukturen. Auch Kopplungen mit sonstigen makromolekularen Verbindungen, wie etwa Polyethylenglykol, können vorgenommen sein. Weitere bevorzugte chemische Modifikationen sind die Modifikation von einer oder mehreren der chemischen Gruppen -COOH, -OH, =NH, -NH₂ -SH zu -COOR, -OR, -NHR, -NR2, -NHR, -NR, -SR; wobei:
R ist-CH=CH-R2, -C≡C-R2, -C(R2)=CH₂, -C(R2)=C(R3), -CH=NR2, -C(R2)=N-R3, ein 4-7 C-Ring System mit oder ohne Substitution, ein 4-7 Stickstoffheterozyklus mit oder ohne Substitution, oder eine C₂ bis C₈ Kette mit 1 bis 5 Doppel- oder Dreifachbindungen mit Substitutionen ausgewählt aus R1, R2, oder R3, wobei
-R1 ist H, -R, -NO₂, -CN, Halogenidsubstituent, -N₃, -C1-8 alkyl, -(CH₂)nCO₂R2, -C2-8 alkenyl-CO₂R2, -O(CH₂)nCO₂R2, -C(O)NR2R3, -P(O)(OR2)₂, alkyl substituiertes tetrazol-5-yl, -(CH₂)nO(CH₂)n aryl, -NR2R3, -(CH₂)n OR2, -(CH₂)n SR2, -N(R2)C(O)R3, -S(O₂)NR2R3, -N(R2)S(O₂)R3, -(CHR2)n NR2R3, -C(O)R3, (CH₂)n N(R3)C(O)R3, -N(R2)CR2R3, substituiertes oder nicht substituiertes (CH₂)n-zykloalkyl, substituiertes oder nicht substituiertes (CH₂)n-phenyl, oder -zyklus; wobei n eine Zahl größer als 1 ist;
-R2 ist H, Halogenidsubstituent, -alkyl, -halogenalkyl, -(CH₂)n-phenyl, -(CH₂)1-3-biphenyl, -(CH₂)1-4-Ph-N(SO₂-C1-2-alkyl)₂, -CO(CHR1)n-OR1, -(CHR1)n-Heterozyklus, -(CHR1)n-NH-CO-R1, -(CHR1)n-NH-SO₂R1, -(CHR1)n-Ph-N(SO₂-C1-2-alkyl)₂, -(CHR1)n-C(O)(CHR1)-NHR1, -(CHR1)n-C(S)(CHR1)-NHR1, -(CH₂)nO(CH₂)nCH₃, -CF₃, -C₂-C₅ acyl, -(CHR1)nOH, -(CHR1)nCO₂R1, -(CHR1)n-O-alkyl, -(CHR1)n-O-(CH₂)n-O-alkyl, -(CHR1)n-S-alkyl, -(CHR1)n-S(O)-alkyl, -(CHR1)n-S(O₂)-alkyl, -(CHR1)n-S(O₂)-NHR3, -(CHR3)n-N₃, -(CHR3)nNHR4, eine C₂ bis C₈ Kette Alken-Kette mit 1 bis 5 Doppelbindungen, eine C₂ bis C₈ Kette Alkin-Kette mit 1 bis 5 Dreifachbindungen, substituierter oder nicht substituierter -(CHR3)n Heterozyklus, substituiertes oder nicht substituiertes gesättigtes oder nicht gesättigtes -(CHR3)n Zykloalkyl; wobei n eine Zahl größer als 1 ist und R1 und R3 gleich oder unterschiedlich sein können;
-R3 ist H, -OH, -CN, substituiertes Alkyl, - C₂ bis C₈ Alkenyl, substituiertes oder nicht substituiertes Zykloalkyl, -N(R1)R2, gesättigter oder nicht gesättigter C₅ bis C₇ Heterozyklus oder Heterobizyklus von 4 bis 7 C-Atomen, -NR1, -NR2, -NR1R2 bestehend aus einem gesättigten oder nicht gesättigten Heterozyklus oder einem Heterobizyklus von 4 bis 7 C-Atomen;
-R4 ist H, -(CH₂)nOH, -C(O)OR5, -C(O)SR5, -(CH₂)n C(O)NR6R7, -O-C(O)-O-R6, eine Aminosäure oder ein Peptid; wobei n eine Zahl zwischen 0 und 4 ist;
-R5 ist H,
-R6 ist -C(R7)-(CH₂)n-O-C(O)-R8, -(CH₂)n-C(R7)-O-C(O)R8, -(CH₂)n-C(R7)-O-C(O)-O-R8, oder -C(R7)-(CH₂)n-O-C(O)-O-R8; wobei n eine Zahl zwischen 0 und 4 ist; und
-R7 und R8 sind jeweils H, Alkyl, substituiertes Alkyl, Aryl, substituiertes Aryl, Alkenyl, substituiertes Alkenyl, Alkinyl, substituiertes Alkinyl, Heterozyklus, substituierter Heterozyklus, Alkylaryl, substituiertes Alkylaryl, Zykloalkyl, substituiertes Zykloalkyl, oder CH₂CO₂alkyl, wobei R7 und R8 gleich oder unterschiedlich sein können.

Erfindungsgemäß ist es weiter möglich, alle möglichen Kombinationen der vorstehend als zugehörig zu i., ii. oder iii. genannten Substanzen und/oder deren Derivate einzusetzen.

Ein erfindungsgemäßes flüssiges Wasch- oder Reinigungsmittel kann als solches oder nach Verdünnen mit Wasser zur Reinigung von Textilien und/oder harten Oberflächen eingesetzt werden. Eine solche Verdünnung kann leicht hergestellt werden, indem eine abgemessene Menge des Mittels in einer weiteren Menge Wasser verdünnt wird in bestimmten Gewichtsverhältnissen von Mittel: Wasser und optional diese Verdünnung geschüttelt wird, um eine gleichmäßige Verteilung des Mittels im Wasser sicherzustellen. Mögliche Gewichts- oder Volumenverhältnisse der Verdünnungen sind von 1:0 Mittel : Wasser bis 1:10000 oder 1:20000 Mittel: Wasser, vorzugsweise von 1:10 bis 1:2000 Mittel: Wasser.

Als flüssige Wasch- oder Reinigungsmittel können hierbei alle flüssigen bzw. fließfähigen Darreichungsformen dienen. "Fließfähig" im Sinne der vorliegenden Anmeldung sind dabei Mittel, welche gießbar sind und Viskositäten bis hin zu mehreren 10.000 mPas aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 5 bis 30000 mPas. Bevorzugte Mittel haben Viskositäten von 10 bis 15000 mPas, wobei Werte zwischen 120 bis 8000 mPas besonders bevorzugt sind. Ein flüssiges Wasch- oder Reinigungsmittel im Rahmen der vorliegenden Erfindung kann daher auch gelförmig oder pastenförmig sein, es kann als homogene Lösung oder Suspension vorliegen, sowie beispielsweise versprühbar oder in sonstigen üblichen Darreichungsformen konfektioniert sein. Zu den Waschmitteln zählen alle denkbaren Waschmittelarten, insbesondere Waschmittel für Textilien, Teppiche oder Naturfasern. Sie können für die manuelle und/oder auch die maschinelle Anwendung vorgesehen sein. Zu den Waschmitteln zählen ferner Waschhilfsmittel, die bei der manuellen oder maschinellen Textil-wäsche zum eigentlichen Waschmittel hinzudosiert werden, um eine weitere Wirkung zu erzielen. Zu den Reinigungsmitteln werden alle, ebenfalls in sämtlichen genannten Darreichungsformen vorkommenden Mittel zur Reinigung und/oder Desinfektion harter Oberflächen, manuelle und maschinelle Geschirrspülmittel, Teppichreiniger, Scheuermittel, Glasreiniger, WC-Duftspüler, usw. gezählt. Textilvor- und Nachbehandlungsmittel sind schließlich auf der einen Seite solche Mittel, mit denen das Wäschestück vor der eigentlichen Wäsche in Kontakt gebracht wird, beispielsweise zum Anlösen hartnäckiger Verschmutzungen, auf der anderen Seite solche, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Desinfektionsmittel sind beispielsweise Händedesinfektionsmittel, Flächendesinfektionsmittel und Instrumentendesinfektionsmittel, die ebenfalls in den genannten Darreichungsformen vorkommen können.

In einer weiteren bevorzugten Ausführungsform der Erfindung ist das Wasch- oder Reinigungsmittel dadurch gekennzeichnet, dass es mindestens einen weiteren Inhaltsstoff umfasst, insbesondere einen, der ausgewählt ist aus der Gruppe bestehend aus Phosphonat, Tensid, Gerüststoff (Builder), nichtwässriges Lösungsmittel, Säure, wasserlösliches Salz, Verdickungsmittel sowie Kombinationen hiervon.

Phosphonate sind Salze und organische Verbindungen, insbesondere Ester, der Phosphonsäure. Als Salze existieren primäre (M'H₂PO₃ bzw. HP(O)(OH)(OM')) und sekundäre (M'₂HPO₃ bzw. HP(O)(OM')₂) Phosphonate, wobei M' für ein einwertiges Metall steht. Diese anorganischen Phosphonate und werden auch als primäre bzw. sekundäre Phosphite bezeichnet. Anorganische Phosphonate entstehen beispielsweise durch Umsetzung von Phosphonsäure HP(O)(OH)₂, insbesondere der stabilen tautomeren Form der Phosphorigsäure mit einem (primäre) oder zwei (sekundäre) Äquivalenten Base, beispielsweise Alkalimetallhydroxid. Im Rahmen der vorliegenden Erfindung bevorzugt sind organisch P-substituierte Phosphonate, die eine Phosphor-Kohlenstoff-Bindung aufweisen (Phosphor-organische Verbindungen). Ihre allgemeine Struktur ist R1P(O)(OR2)₂, mit R1 und/oder R2 = Alkyl, Aryl oder H, wobei die Alkyl- bzw. Arylreste weitere Substitutionen aufweisen oder weitere chemische Gruppen tragen können. Organisch P-substituierte Phosphonate entstehen beispielsweise durch Michaelis-Arbusov-Reaktion. Viele dieser Phosphonate sind löslich in Wasser. Einige technisch wichtige Phosphonate tragen ferner Amino-Gruppe(n) in der Art NR-(CH2)ₓ-PO(OH)₂ (R = Alkyl, Aryl oder H). Einige diese Aminophosphonate haben strukturelle Ähnlichkeiten mit Komplexbildnern wie EDTA, NTA oder DTPA und haben eine ähnliche Funktion. Zu besonders bevorzugten Phosphonaten im Rahmen der vorliegenden Erfindung zählen insbesondere Organophosphonate wie beispielsweise 1-Hydroxyethan-1,1-diphosphonsäure (HEDP), Aminotri(methylenphosphonsäure) (ATMP, auch bezeichnet als Amino-tris(methylenphosphonsäure) bzw. Nitrolotris(methylenphosphonsäure) (NTMP)), Diethylentriamin-penta(methylenphosphonsäure) (DTPMP oder DETPMP oder DTPNT), Ethylendiamintetra(methylenphosphonsäure) (EDTMP, auch bezeichnet als Ethylendiamintetra(methylenphosphonsäure) sowie 2-Phosphonobutan-1,2,4-tricarbonsäure (PBS-AM, auch bezeichnet als 2-Phosphonobutan-1,2,4-tricarbonsäure bzw. 3-Carboxy-3-phosphonoadipinsäure), die zumeist in Form ihrer Ammonium- oder Alkalimetallsalze eingesetzt werden. Besonders bevorzugt ist Diethylentriamin-penta(methylenphosphonsäure)-Natrium. Ein solches Phosphonat ist beispielsweise unter dem Handelsnamen Dequest® 2066 (Firma Thermphos) erhältlich.

Vorzugsweise ist das Phosphonat in einer Menge von 0,01 bis 2,5 Gew.-% und zunehmend bevorzugt von 0,02 bis 2 Gew.-%, von 0,03 bis 1,5 Gew.-% und insbesondere von 0,05 bis 1 Gew.-% in dem Wasch- oder Reinigungsmittel enthalten.

Als Tensid(e) können anionische, nichtionische, zwitterionische und/oder amphotere Tenside eingesetzt werden. Bevorzugt sind aus anwendungstechnischer Sicht Mischungen aus anionischen und nichtionischen Tensiden. Der Gesamttensidgehalt des flüssigen Wasch- oder Reinigungsmittels liegt vorzugsweise unterhalb von 60 Gew.-% und besonders bevorzugt unterhalb von 45 Gew.-%, bezogen auf das gesamte flüssige Wasch- oder Reinigungsmittel.

Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das Wasch-, Reinigungs-, Nachbehandlungs- oder Waschhilfsmittel einen C₁₂₋₁₈-Fettalkohol mit 7 EO oder einen C₁₃₋₁₅-Oxoalkohol mit 7 EO als nichtionisches Tensid.

Der Gehalt an nichtionischen Tensiden beträgt in dem Wasch- oder Reinigungsmittel bevorzugt 3 bis 40 Gew.-%, vorzugsweise 5 bis 30 Gew.-% und insbesondere 7 bis 20 Gew.-%, jeweils bezogen auf das gesamte Wasch- oder Reinigungsmittel.

Neben den nichtionischen Tensiden kann das Wasch- oder Reinigungsmittel auch anionische Tenside enthalten. Als anionisches Tensid werden vorzugsweise Sulfonate, Sulfate, Seifen, Alkylphosphate, anionische Silikontenside und Mischungen daraus eingesetzt.

Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete anionische Tenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Auch bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Magnesium- oder Ammoniumsalze vorliegen. Vorzugsweise liegen die anionischen Tenside in Form ihrer Natriumsalze vor. Weitere bevorzugte Gegenionen für die anionischen Tenside sind auch die protonierten Formen von Cholin, Triethylamin oder Methylethylamin.

Der Gehalt eines Wasch- oder Reinigungsmittels an anionischen Tensiden kann 1 bis 40 Gew.-%, vorzugsweise 5 bis 30 Gew.-% und ganz besonders bevorzugt 10 bis 25 Gew.-%, jeweils bezogen auf das gesamte Wasch- oder Reinigungsmittel, betragen.

Als Gerüststoffe (Builder), die in dem Wasch- oder Reinigungsmittel enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche in dem Wasch- oder Reinigungsmittel vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit einer Molmassen von 1.000 bis 5.000 g / mol bevorzugt in den flüssigen Wasch- oder Reinigungsmitteln eingesetzt.

Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Die erfindungsgemäßen Wasch- oder Reinigungsmittel sind flüssig und enthalten vorzugsweise Wasser als Hauptlösungsmittel. Daneben können dem Wasch- oder Reinigungsmittel nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Di-isopropylenglykolmonomethylether, Di-isopropylenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel. Es ist allerdings bevorzugt, dass das Wasch- oder Reinigungsmittel ein Polyol als nicht-wässriges Lösungsmittel enthält. Das Polyol kann insbesondere Glycerin, 1,2-Propandiol, 1,3-Propandiol, Ethylenglycol, Diethylenglycol und/oder Dipropylenglycol umfassen. Insbesondere bevorzugt enthält das Wasch- oder Reinigungsmittel eine Mischung aus einem Polyol und einem einwertigen Alkohol. Nichtwässrige Lösungsmittel können in dem Wasch- oder Reinigungsmittel in Mengen zwischen 0,5 und 15 Gew.-%, bevorzugt aber unter 12 Gew.-% und eingesetzt werden.

Zur Einstellung eines gewünschten, sich durch die Mischung der übrigen Komponenten nicht von selbst ergebenden pH-Werts können die Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln in Mengen von vorzugsweise nicht über 20 Gew.-%, insbesondere von 1,2 Gew.-% bis 17 Gew.-%, enthalten.

Ein Mittel im Sinne der Erfindung kann weiterhin ein oder mehrere wasserlösliche Salze enthalten, die beispielsweise zur Viskositätseinstellung dienen. Es kann sich dabei um anorganische und/oder organische Salze handeln. Einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben. Einsetzbare organische Salze sind beispielsweise farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Zur Verdickung kann ein erfindungsgemäßes Mittel ein oder mehrere Verdickungsmittel enthalten. Bevorzugt ist das Verdickungsmittel ausgewählt aus der Gruppe umfassend Xanthan, Guar, Carrageenan, Agar-Agar, Gellan, Pektin, Johannisbrotkernmehl und Mischungen daraus. Diese Verbindungen sind auch in Gegenwart von anorganischen Salzen effektive Verdickungsmittel. In einer besonders bevorzugten Ausführungsform enthält das Wasch- oder Reinigungsmittel Xanthan als Verdickungsmittel, da Xanthan auch in Gegenwart von hohen Salzkonzentrationen effektiv verdickt und eine makroskopische Auftrennung der kontinuierlichen Phase verhindert. Zusätzlich stabilisiert das Verdickungsmittel die kontinuierliche, tensidarme Phase und verhindert eine makroskopische Phasenseparation.

Alternativ oder ergänzend können auch (Meth)Acrylsäure(co)polymere als Verdickungsmittel eingesetzt werden. Geeignete Acryl- und Methacryl(co)polymere umfassen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients" der "The Cosmetic, Toiletry and Fragrance Association (CTFA)": Carbomer), die auch als Carboxyvinylpolymere bezeichnet werden. Solche Polyacrylsäuren sind unter anderem unter den Handelsnamen Polygel® und Carbopol® erhältlich. Weiterhin sind beispielsweise folgende Acrylsäure-Copolymere geeignet: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCI Acrylates Copolymer), die beispielsweise unter den Handelsnamen Aculyn®, Acusol® oder Tego® Polymer erhältlich sind; (ii) vernetzte hochmolekulare Acrylsäure-Copolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCI Acrylates/C₁₀₋₃₀ Alkyl Acrylate Crosspolymer) gehören und die beispielsweise unter dem Handelsnamen Carbopol® erhältlich sind. Weitere geeignete Polymere sind (Meth)Acrylsäure(co)polymere des Typs Sokalan®.

Es kann bevorzugt sein, dass das erfindungsgemäße Wasch- oder Reinigungsmittel ein (Meth)Acrylsäure(co)polymer in Kombination mit einem weiteren Verdickungsmittel, vorzugsweise Xanthan, enthält. Das Wasch- oder Reinigungsmittel kann 0,05 bis 1,5 Gew.-% und vorzugsweise 0,1 bis 1 Gew.-%, jeweils bezogen auf das gesamte Wasch- oder Reinigungsmittel, Verdickungsmittel enthalten. Die Menge an eingesetztem Verdickungsmittel ist dabei abhängig von der Art des Verdickungsmittels und dem gewünschten Grad der Verdickung.

Flüssige beziehungsweise pastöse erfindungsgemäße Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

Erfindungsgemäße Wasch- oder Reinigungsmittel können ausschließlich eine Protease und eine Amylase enthalten wie beschrieben. Alternativ können sie auch weitere hydrolytische Enzyme oder andere Enzyme in einer für die Wirksamkeit des Mittels zweckmäßigen Konzentration enthalten. Einen weiteren Gegenstand der Erfindung stellen somit Mittel dar, die ferner eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase, sowie deren Gemische. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1,5 Gew.-%, von 0,00005-1 Gew.-%, von 0,0001 bis 0,75 Gew.-% und besonders bevorzugt von 0,0005 bis 0,5 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der genannten Protease und der Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In einer weiteren Ausführungsform der Erfindung ist das Wasch- oder Reinigungsmittel dadurch gekennzeichnet, dass es ein maschinelles Geschirrspülmittel ist. Als maschinelle Geschirrspülmittel werden nach Maßgabe dieser Anmeldung Zusammensetzungen bezeichnet, die zur Reinigung verschmutzten Geschirrs in einem maschinellen Geschirrspülverfahren eingesetzt werden können. Damit unterscheiden sich die erfindungsgemäßen maschinellen Geschirrspülmittel beispielsweise von den maschinellen Klarspülmitteln, die stets in Kombination mit maschinellen Geschirrspülmitteln eingesetzt werden und keine eigene Reinigungswirkung entfalten. An maschinell gespültes Geschirr werden häufig höhere Anforderungen gestellt als an manuell gespültes Geschirr. So soll das Geschirr nach der maschinellen Reinigung nicht nur frei von Speiseresten sein, sondern beispielsweise auch keine weißlichen, auf Wasserhärte oder anderen mineralischen Salzen beruhenden Flecken aufweisen, die mangels Netzmittel aus eingetrockneten Wassertropfen stammen. Moderne maschinelle Geschirrspülmittel erfüllen diese Anforderungen durch die Integration reinigender und/oder pflegender und/oder wasserenthärtender und/oder klarspülaktiver Wirkstoffe und sind dem Verbraucher beispielsweise als "2in1"- oder "3in1" Geschirrspülmittel bekannt. Als für den Reinigungs- wie für den Klarspülerfolg wesentlichen Bestandteil enthalten die maschinellen Geschirrspülmittel Gerüststoffe. Diese Gerüststoffe erhöhen zum einen die Alkalität der Reinigungsflotte, wobei mit steigender Alkalität Fette und Öle emulgiert und verseift werden, und vermindern zum anderen durch Komplexierung der in der wässrigen Flotte enthaltenen Calciumionen die Wasserhärte der Reinigungsflotte. In einer weiteren bevorzugten Ausgestaltung ist das maschinelle Geschirrspülmittel von einer wasserlöslichen Folie umschlossen. Vorzugsweise umfasst die Folie einen Polyvinylalkohol (PVA) oder besteht aus Polyvinylalkohol (PVA).

Einen weiteren Erfindungsgegenstand stellt die Verwendung eines erfindungsgemäßen Wasch- oder Reinigungsmittels zur Entfernung von Anschmutzungen, insbesondere von protease- und/oder amylase-sensitiven Anschmutzungen, auf Textilien oder harten Oberflächen, d.h. zur Reinigung von Textilien oder von harten Oberflächen, dar. Denn erfindungsgemäße Mittel können, insbesondere auf Grund der enthaltenen Kombination von Protease und Amylase, vorteilhaft dazu verwendet werden, um von Textilien oder von harten Oberflächen entsprechende Verunreinigungen zu beseitigen. Ausführungsformen dieses Erfindungsgegenstandes stellen beispielsweise die Handwäsche, die manuelle Entfernung von Flecken von Textilien oder von harten Oberflächen oder die Verwendung im Zusammenhang mit einem maschinellen Verfahren dar. Alle Sachverhalte, Gegenstände und Ausführungsformen, die für erfindungsgemäße Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

Einen weiteren Erfindungsgegenstand stellt ein Verfahren zur Reinigung von Textilien oder von harten Oberflächen dar, wobei in mindestens einem Verfahrensschritt ein erfindungsgemäßes Wasch- oder Reinigungsmittel angewendet wird.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung bzw. Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels bereichert werden und stellen dann Ausführungsformen der vorliegenden Erfindung dar. Alle Sachverhalte, Gegenstände und Ausführungsformen, die für erfindungsgemäße Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

In einer bevorzugten Ausführungsform ist das Verfahren dadurch gekennzeichnet, dass die Amylase in der Waschflotte in einer Konzentration von 1 x 10⁻¹⁰-0,2 Gew.-%, von 0,000001-0,12 Gew.-%, von 0,000005-0,04 Gew.-%, von 0,00001 bis 0,03 Gew.-% und besonders bevorzugt von 0,00005 bis 0,02 Gew.-% vorliegt, und/oder dass die Protease in der Waschflotte in einer Konzentration von 1 x 10⁻¹⁰-0,2 Gew.-%, von 0,000001-0,12 Gew.-%, von 0,000005-0,04 Gew.-%, von 0,00001 bis 0,03 Gew.-% und besonders bevorzugt von 0,00005 bis 0,02 Gew.-% vorliegt, wobei die Angaben auf Aktivprotein in der Waschflotte bezogen sind. In einer weiteren bevorzugten Ausführungsform ist das Verfahren dadurch gekennzeichnet, dass es bei einer Temperatur zwischen 10°C und 60°C, bevorzugt zwischen 20°C und 50°C und besonders bevorzugt zwischen 30°C und 50°C durchgeführt wird.

In erfindungsgemäßen Mitteln eingesetzte Proteasen sind entsprechend der vorstehenden Ausführungen vorteilhaft in erfindungsgemäßen Wasch- und Reinigungsmitteln sowie Verfahren, insbesondere Wasch- und Reinigungsverfahren, einsetzbar. Sie können also vorteilhaft dazu verwendet werden, um in entsprechenden Mitteln eine proteolytische Aktivität bereitzustellen.

Einen weiteren Gegenstand der Erfindung bildet daher die Verwendung einer Protease, die eine Aminosäuresequenz umfasst, die zu der in SEQ ID NO. 1 angegebenen Aminosäuresequenz über deren Gesamtlänge zu mindestens 70% identisch ist und in der Zählung gemäß SEQ ID NO. 1 die Aminosäuresubstitution R99E oder R99D in Kombination den Aminosäuresubstitutionen S3T, V4I und V199I aufweist, zur Bereitstellung einer proteolytischen Aktivität in einem flüssigen Wasch- oder Reinigungsmittel, welches ferner eine Amylase gemäß Anspruch 1 umfasst.

Alle Sachverhalte, Gegenstände und Ausführungsformen, die für erfindungsgemäße Wasch- oder Reinigungsmittel beschrieben sind, sind auch auf die genannten Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.

### Beispiel: Ermittlung der Lagerstabilität eines erfindungsgemäßen flüssigen maschinellen Geschirrspülmittels

Als Basisrezeptur diente ein amylasehaltiges zweiphasiges flüssiges maschinelles Geschirrspülmittel, das wie folgt zusammengesetzt war (alle Angaben in Gewichts-Prozent):

### (a) Enzymphase:

| | |
|---|---|
| Builder | 18,0 |
| Zuckeralkohol | 12,0 |
| Nichtionisches Tensid (C8-C10 Fettalkoholethoxylat mit 22 EO) | 5,0 |
| Alkaliverbindung (Base) | 3,5 |
| Borsäure | 3,0 |
| Phosphonat (HEDP) | 1,5 |
| Amylase | 1,2 |
| Ca-Salz | 1,2 |
| Zn-Salz | 0,2 |
| Verdicker | 1,0 |
| Farbstoff, Parfüm, Konservierungsmittel | 0,3 |
| Wasser | ad 97 |

Als Amylase enthalten war eine α-Amylase-Variante, die gegenüber der α-Amylase AA560 gemäß SEQ ID NO. 4 folgende Sequenzveränderungen in der Zählung der α-Amylase AA560 aufweist: R118K, D183* (Deletion), G184* (Deletion), N195F, R320K, R458K (Firma Novozymes).

### (b) Alkalische Phase:

| | |
|---|---|
| Builder | 12,0 |
| Natriumcarbonat | 10,0 |
| Sulfopolymer | 7,0 |
| Alkaliverbindung (Base) | 4,0 |
| Monoethanolamin | 3,5 |
| Phosphonat (HEDP) | 4,0 |
| Verdicker | 1,0 |
| Farbstoff, Parfüm, Konservierungsmittel | 0,3 |
| Wasser | ad 100 |

Die Enzymphase der Basisrezeptur wurde für die verschiedenen Versuchsansätze jeweils mit 3 Gew.-% von Präparationen der folgenden Proteasen versetzt (resultierend in jeweils 0,5 Gew.-% Aktivprotein):
Ansatz 1: Leistungsverbesserte Variante der Protease aus Bacillus lentus gemäß SEQ ID NO. 2 der WO2011/032988 (Referenz);
Ansatz 2: Protease gemäß SEQ ID NO. 2 (SEQ ID NO. 1 + S3T + V4I + R99E + V199I).

Zur Ermittlung der Reinigungsleistung wurden beide Phasen zu gleichen Teilen dosiert (jeweils 20g pro Phase). Gewaschen wurde in einem pH-Wertebereich zwischen pH 9 und pH 10 in einer Geschirrspülmaschine G698SC der Firma Miele in einem Volumen von 4 Litern für eine Dauer von 60 Minuten bei einer Temperatur von 50°C.

Es wurde Geschirr mit folgenden Anschmutzungen wurden verwendet: Milch (A), Hackfleisch (B), Eigelb (C), Haferflocken (D) und Stärke (E).

Die Auswertung der Reinigungsleistung erfolgt gemäß der Standard IKW-Methode visuell anhand einer Skala von 1 bis 10, wobei der Wert 10 die beste Note ist (kein erkennbarer Rückstand).

Die Waschmittel der Ansätze 1 und 2 wurden hinsichtlich ihrer Reinigungsleistung vor und nach Lagerung von vier Wochen bei 40°C überprüft. Die Ergebnisse sind in nachstehender Tabelle 1 zusammengefasst:

**Tabelle 1:**

| Anschmutzung | A | B | C | D | E |
|---|---|---|---|---|---|
| Ansatz 1 vor Lagerung | 5,6 | 10,0 | 5,2 | 7,7 | 9,6 |
| Ansatz 2 vor Lagerung | 5,2 | 10,0 | 5,7 | 7,8 | 9,8 |
| Ansatz 1 nach Lagerung | 5,1 | 2,8 | 1,7 | 2,0 | 3,4 |
| Ansatz 2 nach Lagerung | 5,2 | 7,7 | 3,0 | 5,1 | 4,0 |

Nach einer Lagerung von vier Wochen bei 40°C zeigt sich deutlich, dass die erfindungsgemäße Zusammensetzung - bewirkt durch die enthaltene Protease - eine deutlich verbesserte Reinigungsleistung zeigt, insbesondere an den protease-sensitiven Anschmutzungen B und C (proteolytische Reinigungsleistung). Insbesondere ist auch die Reinigungsleistung an den amylase-sensitiven Anschmutzungen D und E verbessert (amylolytische Reinigungsleistung).

## Patentansprüche

1. Flüssiges Wasch- oder Reinigungsmittel umfassend
(a) eine Protease, die eine Aminosäuresequenz umfasst, die zu der in SEQ ID NO. 1 angegebenen Aminosäuresequenz über deren Gesamtlänge zu mindestens 70% identisch ist und in der Zählung gemäß SEQ ID NO. 1 die Aminosäuresubstitution R99E oder R99D in Kombination mit den Aminosäuresubstitutionen S3T, V4I und V199I aufweist, und
(b) eine Amylase, **dadurch gekennzeichnet, dass** die Amylase eine α-Amylase-Variante der α-Amylase AA560 gemäß SEQ ID NO. 4 ist, die
(i) gegenüber der α-Amylase AA560 gemäß SEQ ID NO. 4 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K, oder
(ii) gegenüber der α-Amylase AA560 gemäß SEQ ID NO. 4 folgende Sequenzveränderungen in der Zählung der α-Amylase AA560 aufweist:
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M202I / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V214T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V214I / Y295F / N299Y / M323T / A339S,
(35) M9L / G149A / G182T / G186A / M202I / T257I / Y295F / N299Y / M323T / A339S / E345R,
(36) M9L / G149A / M202I / V214T / Y295F / N299Y / M323T / A339S / E345R / N471E,
(37) M9L / G149A / M202L / V214I / Y295F / M323T / A339S / E345R/ / M382Y / N471E,
(38) M9L / G149A / G182T / G186A / M202I / V214I / Y295F / N299Y / M323T / A339S / N471E,
(39) M9L / G149A / G182T / G186A / M202L / T257I / Y295F / N299Y / M323T / A339S / E345R / N471E,
(40) M202L / M105F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R,
(51) M202L / K446R / N484Q,
(52) M202I / Y295F,
(53) M202I / A339S,
(54) M202I / M105F / M208F,
(55) G133E / M202I / Q361E,
(56) G133E / M202I / R444E,
(57) M202I / M323T,
(58) M202I / M323T / M309L,
(59) M202I / M323T / M430I,
(60) M202I / V214T / R444Y,
(61) M202I / N283D / Q361E,
(62) M202I / M382Y / K383R,
(63) M202I / K446R / N484Q,
(64) M202V / M105F / M208F,
(65) G133E / M202V / Q361E,
(66) G133E / M202V / R444E.
(67) M202V / M323T,
(68) M202V / M323T / M309L,
(69) M202V / M323T / M430I,
(70) M202V / M323T / M9L,
(71) M202V / V214T / R444Y,
(72) M202V / N283D / Q361E,
(73) M202V / M382Y / K383R,
(74) M202V / K446R / N484Q,
(75) M202T / M105F / M208F,
(76) G133E / M202T / Q361E,
(77) G133E / M202T / R444E,
(78) M202T / Y295F,
(79) M202T / A339S,
(80) M202T / M323T,
(81) M202T / M323T / M309L,
(82) M202T / M323T / M430I,
(83) M202T / M323T / M9L,
(84) M202T / V214T / R444Y,
(85) M202T / N283D / Q361E,
(86) M202T / A339S,
(87) M202T / Y295F
(88) M202T / N299F,Y,
(89) M202T / M382Y / K383R oder
(90) M202T / K446R / N484Q

2. Wasch- oder Reinigungsmittel nach Anspruch 1, **dadurch gekennzeichnet, dass** die Protease eine Aminosäuresequenz umfasst, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist. , insbesondere eine Protease, die eine Aminosäuresequenz gemäß SEQ ID NO. 2 aufweist.

3. Wasch- oder Reinigungsmittel nach Anspruch 1, **dadurch gekennzeichnet, dass** die Protease eine Aminosäuresequenz umfasst, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist, insbesondere eine Protease, die eine Aminosäuresequenz gemäß SEQ ID NO. 3 aufweist.

4. Wasch- oder Reinigungsmittel nach einem der Ansprüche 1 bis 3, **dadurch gekennzeichnet, dass** die Amylase eine α-Amylase-Variante der α-Amylase AA560 gemäß SEQ ID NO. 4 ist, die eine, zwei, drei, vier, fünf oder sechs der folgenden Sequenzveränderungen in der Zählung gemäß der α-Amylase AA560 aufweist: R118K, D183* (Deletion), G184* (Deletion), N195F, R320K, R458K.

5. Wasch oder Reinigungsmittel nach einem der Ansprüche 1 bis 4, **dadurch gekennzeichnet, dass** die Amylase in einer Menge von 1 x 10⁻⁸ bis 5 Gewichts-Prozent, bezogen auf aktives Protein, enthalten ist, und/oder dass die Protease in einer Menge von 1 x 10⁻⁸ bis 5 Gewichts-Prozent, bezogen auf aktives Protein, enthalten ist.

6. Wasch- oder Reinigungsmittel nach einem der Ansprüche 1 bis 5, **dadurch gekennzeichnet, dass** es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

7. Wasch- oder Reinigungsmittel nach einem der Ansprüche 1 bis 6, **dadurch gekennzeichnet, dass** es mindestens einen weiteren Inhaltsstoff umfasst, insbesondere einen, der ausgewählt ist aus der Gruppe bestehend aus Phosphonat, Tensid, Gerüststoff (Builder), nichtwässriges Lösungsmittel, Säure, wasserlösliches Salz, Verdickungsmittel sowie Kombinationen hiervon.

8. Wasch- oder Reinigungsmittel nach einem der Ansprüche 1 bis 7, **dadurch gekennzeichnet, dass** es mindestens ein weiteres Enzym umfasst, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine Lipase, sowie deren Gemische.

9. Wasch- oder Reinigungsmittel nach einem der Ansprüche 1 bis 8, **dadurch gekennzeichnet, dass** es ein maschinelles Geschirrspülmittel ist.

10. Verwendung eines Wasch- oder Reinigungsmittels nach einem der Ansprüche 1 bis 9 zur Entfernung von protease-sensitiven Anschmutzungen auf Textilien oder harten Oberflächen.

11. Verfahren zur Reinigung von Textilien oder harten Oberflächen, **dadurch gekennzeichnet, dass** in mindestens einem Verfahrensschritt ein Wasch- oder Reinigungsmittel nach einem der Ansprüche 1 bis 9 angewendet wird.

12. Verfahren nach Anspruch 11, **dadurch gekennzeichnet, dass** die Amylase in der Waschflotte in einer Konzentration von 1 x 10⁻¹⁰-0,2 Gew.-% vorliegt, und/oder dass die Protease in der Waschflotte in einer Konzentration von 1 x 10⁻¹⁰-0,2 Gew.-% vorliegt.

13. Verfahren nach Anspruch 11 oder 12, **dadurch gekennzeichnet, dass** es bei einer Temperatur zwischen 10°C und 60°C, bevorzugt zwischen 20°C und 50°C und besonders bevorzugt zwischen 30°C und 50°C durchgeführt wird.

14. Verwendung einer Protease, die eine Aminosäuresequenz umfasst, die zu der in SEQ ID NO. 1 angegebenen Aminosäuresequenz über deren Gesamtlänge zu mindestens 70% identisch ist und in der Zählung gemäß SEQ ID NO. 1 die Aminosäuresubstitution R99E oder R99D in Kombination mit den Aminosäuresubstitutionen S3T, V4I und V199I aufweist,
zur Bereitstellung einer proteolytischen Aktivität in einem flüssigen Wasch- oder Reinigungsmittel, welches eine Amylase umfasst, die eine α-Amylase-Variante der α-Amylase AA560 gemäß SEQ ID NO. 4 ist, wie
(i) gegenüber der α-Amylase AA560 gemäß SEQ ID NO. 4 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K, oder
(ii) gegenüber der α-Amylase AA560 gemäß SEQ ID NO. 4 folgende Sequenzveränderungen in der Zählung der α-Amylase AA560 aufweist:
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M202I / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V24T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V214I / Y295F / N299Y / M323T / A339S,
(35) M9L / G149A / G182T / G186A / M202I / T257I / Y295F / N299Y / M323T / A339S / E345R,
(36) M9L / G149A / M202I / V214T / Y295F / N299Y / M323T / A339S / E345R / N471E,
(37) M9L / G149A / M202L / V214I / Y295F / M323T / A339S / E345R / M382Y / N471E,
(38) M9L / G149A / G182T / G186A / M202I / V214I / Y295F / N299Y / M323T / A339S / N471E,
(39) M9L / G149A / G182T / G186A / M202L / T257I / Y295F / N299Y / M323T / A339S / E345R / N471E,
(40) M202L / ML05F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R,
(51) M202L / K446R / N484Q,
(52) M202I / Y295F,
(53) M202I / A339S,
(54) M202I / M105F / M208F,
(55) G133E / M202I / Q361E,
(56) G133E / M202I / R444E,
(57) M202I / M323T,
(58) M202I / M323T / M309L,
(59) M202I / M323T / M430I,
(60) M202I / V214T / R444Y,
(61) M202I / N283D / Q361E,
(62) M202I / M382Y / K383R,
(63) M202I / K446R / N484Q,
(64) M202V / M105F / M208F,
(65) G133E / M202V / Q361E,
(66) G133E / M202V / R444E,
(67) M202V / M323T,
(68) M202V / M323T / M309L,
(69) M202V / M323T / M430I,
(70) M202V / M323T / M9L,
(71) M202V / V214T / R444Y,
(72) M202V / N283D / Q361E,
(73) M202V / M382Y / K383R,
(74) M202V / K446R / N484Q,
(75) M202T / M105F / M208F,
(76) G133E / M202T / Q361E,
(77) G 133E / M202T / R444E,
(78) M202T / Y295F,
(79) M202T / A339S,
(80) M202T / M323T,
(81) M202T / M323T / M309L,
(82) M202T / M323T / M430I,
(83) M202T / M323T / M9L,
(84) M202T / V214T / R444Y,
(85) M202T / N283D / Q361E,
(86) M202T / A339S,
(87) M202T / Y295F
(88) M202T / N299F,Y,
(89) M202T / M382Y / K383R oder
(90) M202T / K446R / N484Q

## Claims

1. A liquid washing or cleaning agent comprising
(a) a protease which comprises an amino acid sequence which, over the entire length thereof, is at least 70% identical to the amino acid sequence stated in SEQ ID NO 1 and, in the numbering according to SEQ ID NO 1, has the amino acid substitution R99E or R99D in combination with amino acid substitutions S3T, V4I and V199I, and
(b) an amylase, **characterised in that** the amylase is an α-amylase variant of α-amylase AA560 according to SEQ ID NO 4 which,
(i) relative to the α-amylase AA560 according to SEQ ID NO 4, has one, two, three, four, five or six of the following sequence modifications in the numbering of α-amylase AA560: R118K, D183* (deletion), G184* (deletion), N195F, R320K, R458K, or,
(ii) relative to the α-amylase AA560 according to SEQ ID NO 4, has the following sequence modifications in the numbering of α-amylase AA560:
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M202I / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V214T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V214I / Y295F / N299Y / M323T / A339S,
(35) M9L / G149A / G182T / G186A / M202I / T257I / Y295F / N299Y / M323T / A339S / E345R,
(36) M9L / G149A / M202I / V214T / Y295F / N299Y / M323T / A339S / E345R / N471E,
(37) M9L / G149A / M202L / V214I / Y295F / M323T / A339S / E345R / M382Y / N471E,
(38) M9L / G149A / G182T / G186A / M202I / V214I / Y295F / N299Y / M323T / A339S / N471E,
(39) M9L / G149A / G182T / G186A / M202L / T257I / Y295F / N299Y / M323T / A339S / E345R / N471E,
(40) M202L / M105F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R,
(51) M202L / K446R / N484Q,
(52) M202I / Y295F,
(53) M202I / A339S,
(54) M202I / M105F / M208F,
(55) G133E / M202I / Q361E,
(56) G133E / M202I / R444E,
(57) M202I / M323T,
(58) M202I / M323T / M309L,
(59) M202I / M323T / M430I,
(60) M202I / V214T / R444Y,
(61) M202I / N283D / Q361E,
(62) M202I / M382Y / K383R,
(63) M202I / K446R / N484Q,
(64) M202V / M105F / M208F,
(65) G133E / M202V / Q361E,
(66) G133E / M202V / R444E,
(67) M202V / M323T,
(68) M202V / M323T / M309L,
(69) M202V / M323T / M4301,
(70) M202V / M323T / M9L,
(71) M202V / V214T / R444Y,
(72) M202V / N283D / Q361E,
(73) M202V / M382Y / K383R,
(74) M202V / K446R / N484Q,
(75) M202T / M105F / M208F,
(76) G133E / M202T / Q361E,
(77) G133E / M202T / R444E,
(78) M202T / Y295F,
(79) M202T / A339S,
(80) M202T / M323T,
(81) M202T / M323T / M309L,
(82) M202T / M323T / M430I,
(83) M202T / M323T / M9L,
(84) M202T / V214T / R444Y,
(85) M202T / N283D / Q361E,
(86) M202T / A339S,
(87) M202T / Y295F
(88) M202T / N299F,Y,
(89) M202T / M382Y / K383R or
(90) M202T / K446R / N484Q.

2. A washing or cleaning agent according to claim 1, **characterised in that** the protease comprises an amino acid sequence which, over the entire length thereof, is at least 71%, 72%, 73%, 74%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98% and 98.5% identical to the amino acid sequence stated in SEQ ID NO 1, in particular a protease which has an amino acid sequence according to SEQ ID NO 2.

3. A washing or cleaning agent according to claim 1, **characterised in that** the protease comprises an amino acid sequence which, over the entire length thereof, is at least 71%, 72%, 73%, 74%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98% and 98.5% identical to the amino acid sequence stated in SEQ ID NO 1, in particular a protease which has an amino acid sequence according to SEQ ID NO 3.

4. A washing or cleaning agent according to one of claims 1 to 3, **characterised in that** the amylase is an α-amylase variant of the α-amylase AA560 according to SEQ ID NO 4 which has one, two, three, four, five or six of the following sequence modifications in the numbering according to α-amylase AA560: R118K, D183* (deletion), G184* (deletion), N195F, R320K, R458K.

5. A washing or cleaning agent according to one of claims 1 to 4, **characterised in that** the amylase is present in a quantity of 1×10⁻⁸ to 5 weight percent, relative to active protein, and/or **in that** the protease is present in a quantity of 1×10⁻⁸ to 5 weight percent, relative to active protein.

6. A washing or cleaning agent according to one of claims 1 to 5, **characterised in that** it furthermore comprises a component which is selected from
i. an anionic and/or polyanionic substance, and/or
ii. a cationic and/or polycationic substance, and/or
iii. a substance comprising hydroxyl and/or polyhydroxyl group(s).

7. A washing or cleaning agent according to one of claims 1 to 6, **characterised in that** it comprises at least one further ingredient, in particular an ingredient which is selected from the group consisting of phosphonate, surfactant, builder, nonaqueous solvent, acid, water-soluble salt, thickener and combinations hereof.

8. A washing or cleaning agent according to one of claims 1 to 7, **characterised in that** it contains at least one further enzyme, in particular a protease, amylase, cellulase, hemicellulase, mannanase, tannase, xylanase, xanthanase, xyloglucanase, β-glucosidase, pectinase, carrageenase, perhydrolase, oxidase, oxidoreductase or a lipase, and mixtures thereof.

9. A washing or cleaning agent according to one of claims 1 to 8, **characterised in that** it is an automatic dishwashing agent.

10. Use of a washing or cleaning agent according to one of claims 1 to 9 for removing protease-sensitive soiling from textiles or hard surfaces.

11. A method for cleaning textiles or hard surfaces, **characterised in that** the washing or cleaning agent according to one of claims 1 to 9 is used in at least one method step.

12. A method according to claim 11, **characterised in that** the amylase is present in the washing liquor in a concentration of 1×10⁻¹⁰ to 0.2 wt.%, and/or **in that** the protease is present in the washing liquor in a concentration of 1×10⁻¹⁰ to 0.2 wt.%.

13. A method according to claim 11 or claim 12, **characterised in that** it is carried out at a temperature of between 10°C and 60°C, preferably between 20°C and 50°C and more preferably between 30°C and 50°C.

14. Use of a protease which comprises an amino acid sequence which, over the entire length thereof, is at least 70% identical to the amino acid sequence stated in SEQ NO 1 and, in the numbering according to SEQ ID NO 1, has the amino acid substitution R99E or R99D in combination with amino acid substitutions S3T, V41 and V199I, in order to provide a proteolytic activity in a liquid washing or cleaning agent which comprises an amylase which is an α-amylase variant of α-amylase AA560 according to SEQ ID NO 4 which,
(i) relative to the α-amylase AA560 according to SEQ ID NO 4, has one, two, three, four, five or six of the following sequence modifications in the numbering of α-amylase AA560: R118K, D183* (deletion), G184* (deletion), N195F, R320K, R458K, or,
(ii) relative to the α-amylase AA560 according to SEQ ID NO 4, has the following sequence modifications in the numbering of α-amylase AA560:
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M202I / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V214T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V214I / Y295F / N299Y / M323T / A339S,
(35) M9L / G149A / G182T / G186A / M202I / T257I / Y295F / N299Y / M323T / A339S / E345R,
(36) M9L / G149A / M202I / V214T / Y295F / N299Y / M323T / A339S / E345R / N471 E,
(37) M9L / G149A / M202L / V214I / Y295F / M323T / A339S / E345R / M382Y / N471E,
(38) M9L / G149A / G182T / G186A / M202I / V214I / Y295F / N299Y / M323T / A339S / N471E,
(39) M9L / G149A / G182T / G186A / M202L / T257I / Y295F / N299Y / M323T / A339S / E345R / N471 E,
(40) M202L / M105F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R,
(51) M202L / K446R / N484Q,
(52) M2021 / Y295F,
(53) M2021 / A339S,
(54) M202I / M105F / M208F,
(55) G133E / M202I / Q361E,
(56) G133E / M202I / R444E,
(57) M202I / M323T,
(58) M202I / M323T / M309L,
(59) M202I / M323T / M430I,
(60) M202I / V214T / R444Y,
(61) M202I / N283D /Q361E,
(62) M202I / M382Y / K383R,
(63) M202I / K446R / N484Q,
(64) M202V / M105F / M208F,
(65) G133E / M202V / Q361E,
(66) G133E / M202V / R444E,
(67) M202V / M323T,
(68) M202V / M323T / M309L,
(69) M202V / M323T / M430I,
(70) M202V / M323T / M9L,
(71) M202V / V214T / R444Y,
(72) M202V / N283D / Q361E,
(73) M202V / M382Y / K383R,
(74) M202V / K446R / N484Q,
(75) M202T / M105F / M208F,
(76) G133E / M202T / Q361 E,
(77) G133E / M202T / R444E,
(78) M202T / Y295F,
(79) M202T / A339S,
(80) M202T / M323T,
(81) M202T / M323T / M309L,
(82) M202T / M323T / M430I,
(83) M202T / M323T / M9L,
(84) M202T / V214T / R444Y,
(85) M202T / N283D / Q361 E,
(86) M202T / A339S,
(87) M202T / Y295F
(88) M202T / N299F,Y,
(89) M202T / M382Y / K383R or
(90) M202T / K446R / N484Q.

## Revendications

1. Agent de lavage ou de nettoyage liquide, comprenant
(a) une protéase comprenant une séquence d'acides aminés présentant une identité d'au moins 70 % par rapport à la séquence d'acides aminés indiquée dans la SEO ID NO 1 sur l'intégralité de la longueur de cette dernière, et comportant les substitutions d'acides aminés R99E ou R99D en association avec les substitutions d'acides aminés S3T, V4I et V199I selon la numérotation dans la SEQ ID NO 1, et
(b) une amylase, **caractérisé en ce que** ladite amylase est une variante d'alpha-amylase de l'alpha-amylase AA560 selon la SEQ ID NO 4 et laquelle comporte,
(i) par rapport à l'alpha-amylase AA560 selon la SEQ ID NO 4, une, deux, trois, quatre, cinq ou six des modifications de séquence suivantes selon la numérotation dans l'alpha-amylase AA560 :
R118K, D183* (délétion), G184* (délétion), N195F, R320K, R458K,ou
(ii) par rapport à l'alpha-amylase AA560 selon la SEQ ID NO 4, les modifications de séquence suivantes selon la numérotation dans l'alpha-amylase AA560 :
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M2021 / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V214T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V2141 / Y295F / N299Y / M323T / A339S,
(35) M9L / G149A / G182T / G186A / M202I / T257I / Y295F / N299Y / M323T / A339S / E345R
(36) M9L / G149A / M202I / V214T / Y295F / N299Y / M323T / A339S / E345R / N471 E,
(37) M9L / G149A / M202L / V214I / Y295F / M323T / A339S / E345R / M382Y / N471 E,
(38) M9L / G149A / G182T / G186A / M202I / V214I / Y295F / N299Y / M323T / A339S / N471 E,
(39) M9L / G149A / G182T / G186A / M202L / T257I / Y295F / N299Y / M323T / A339S / E345R / N471E,
(40) M202L / M105F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R.
(51) M202L / K446R / N4840,
(52) M202I / Y295F,
(53) M202I / A339S,
(54) M202I / M105F / M208F,
(55) G133E / M202I / 0361E,
(56) G133E / M202I / R444E,
(57) M202I / M323T,
(58) M202I / M323T / M309L,
(59) M202I / M323T / M4301,
(60) M202I / V214T / R444Y,
(61) M202I / N283D / Q361E,
(62) M202I / M382Y / K383R,
(63) M202I / K446R / N484Q,
(64) M202V / M105F / M208F,
(65) G133E / M202V / 0361E,
(66) G133E / M202V / R444E,
(67) M202V / M323T,
(68) M202V / M323T / M309L,
(69) M202V / M323T / M430I,
(70) M202V / M323T / M9L,
(71) M202V / V214T / R444Y,
(72) M202V / N283D 0361 E,
(73) M202V / M382Y / K383R,
(74) M202V / K446R / N4840,
(75) M202T / M105F / M208F,
(76) G133E / M202T / 0361E,
(77) G133E / M202T / R444E,
(78) M202T / Y295F,
(79) M202T / A339S,
(80) M202T / M323T,
(81) M202T / M323T / M309L,
(82) M202T / M323T / M4301,
(83) M202T / M323T / M9L,
(84) M202T / V214T / R444Y,
(85) M202T / N283D / 0361E,
(86) M202T / A339S,
(87) M202T / Y295F
(88)) M202T / N299F,Y,
(89) M202T / M382Y / K383R ou
(90) M202T / K446R / N4840

2. Agent de lavage ou de nettoyage selon la revendication 1, **caractérisé en ce que** ladite protéase comprend une séquence d'acides aminés présentant une identité d'au moins 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% et 98,5% par rapport à la séquence d'acides aminés indiquée dans la SEQ ID NO 1 sur l'intégralité de la longueur de cette dernière, s'agissant notamment d'une protéase comportant une séquence d'acides aminés selon la SEQ ID NO 2.

3. Agent de lavage ou de nettoyage selon la revendication 1, **caractérisé en ce que** ladite protéase comprend une séquence d'acides aminés présentant une identité d'au moins 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% et 98,5% par rapport à la séquence d'acides aminés indiquée dans la SEQ ID NO 1 sur l'intégralité de la longueur de cette dernière, s'agissant notamment d'une protéase comportant une séquence d'acides aminés selon la SEQ ID NO 3.

4. Agent de lavage ou de nettoyage selon l'une des revendications 1 à 3, **caractérisé en ce que** ladite amylase est une variante d'alpha-amylase de l'alpha-amylase AA560 selon la SEQ ID NO 4 et laquelle comporte une, deux, trois, quatre, cinq ou six des modifications de séquence suivantes selon la numérotation dans l'alpha-amylase AA560 : R118K, D183* (délétion), G184* (délétion), N195F, R320K, R458K.

5. Agent de lavage ou de nettoyage selon l'une des revendications 1 à 4, **caractérisé en ce qu'**il contient ladite amylase dans une quantité de 1 x 10⁻⁸ à 5 pour cent en poids, par rapport aux protéines actives et/ou qu'il contient ladite protéase dans une quantité de 1 x 10⁻⁸ à 5 pour cent en poids, par rapport aux protéines actives.

6. Agent de lavage ou de nettoyage selon l'une des revendications 1 à 5, **caractérisé en ce qu'**il comprend en outre un composant choisi parmi
i. une substance anionique et/ou polyanionique, et/ou
ii. une substance cationique et/ou polycationique, et/ou
iii une substance comportant des groupes hydroxyle et/ou polyhydroxyle.

7. Agent de lavage ou de nettoyage selon l'une des revendications 1 à 6, **caractérisé en ce qu'**il comprend au moins un autre ingrédient, ce dernier étant notamment choisi dans le groupe constitué de phosphonates, agents tensioactifs, adjuvants de lavage (builders), solvants non-aqueux, acides, sels hydrosolubles, épaississants ainsi que de leurs combinaisons.

8. Agent de lavage ou de nettoyage selon l'une des revendications 1 à 7, **caractérisé en ce qu'**il comprend au moins une autre enzyme, s'agissant notamment d'une protéase, amylase, cellulase, hémicellulase, mannanase, tannase, xylanase, xanthanase, xyloglucanase, β-glucosidase, pectinase, carraghénase, perhydrolase, oxydase, oxydoréductase ou d'une lipase, ainsi que de leurs mélanges.

9. Agent de lavage ou de nettoyage selon l'une des revendications 1 à 8, **caractérisé en ce qu'**il s'agit d'un produit pour lave-vaisselle.

10. Utilisation d'un agent de lavage ou de nettoyage selon l'une des revendications 1 à 9 pour enlever des salissures sensibles aux protéases se trouvant sur des textiles ou sur des surfaces dures.

11. Procédé de nettoyage de textiles ou de surfaces dures, **caractérisé en ce que**, dans au moins une étape dudit procédé, on met en oeuvre un agent de lavage ou de nettoyage selon l'une des revendications 1 à 9.

12. Procédé selon la revendication 11, **caractérisé en ce que** ladite amylase est présente dans le bain de lavage dans une concentration de 1 x 10⁻¹⁰ à 0,2 % en poids, et/ou que ladite protéase est présente dans le bain de lavage dans une concentration de 1 x 10⁻¹⁰ à 0,2 % en poids.

13. Procédé selon la revendication 11 ou 12, **caractérisé en ce qu'**il est mis en oeuvre à une température entre 10 °C et 60 °C, de préférence entre 20 °C et 50 °C et, avec une préférence particulière, entre 30 °C et 50 °C.

14. Utilisation d'une protéase comprenant une séquence d'acides aminés présentant une identité d'au moins 70 % par rapport à la séquence d'acides aminés indiquée dans la SEQ ID NO 1 sur l'intégralité de la longueur de cette dernière, et comportant les substitutions d'acides aminés R99E ou R99D en association avec les substitutions d'acides aminés S3T, V4I et V199I, selon la numérotation dans la SEQ ID NO 1 pour ainsi fournir une activité protéolytique dans un agent de lavage ou de nettoyage liquide comprenant une amylase qui est une variante d'alpha-amylase de l'alpha-amylase AA560 selon la SEQ ID NO 4 et laquelle comporte,
(i) par rapport à l'alpha-amylase AA560 selon la SEQ ID NO 4, une, deux, trois, quatre, cinq ou six des modifications de séquence suivantes selon la numérotation dans l'alpha-amylase AA560 : R118K, D183* (délétion), G184* (délétion), N195F, R320K, R458K,ou
(ii) par rapport à l'alpha-amylase AA560 selon la SEQ ID NO 4, les modifications de séquence suivantes selon la numérotation dans l'alpha-amylase AA560 :
(1) M9L / M202I,
(2) M9L / M202I / M323T,
(3) M9L / M202I / M323T / M382Y,
(4) M9L / M202I / Y295F / A339S,
(5) M9L / M202I / Y295F,
(6) M9L / M202I / A339S,
(7) M9L / M202I / Y295F / A339S,
(8) M9L / M202I / Y295F / A339S / E345R,
(9) M9L / G149A / M202I / Y295F / A339S / E345R,
(10) M9L / M202L,
(11) M9L / M202L / M323T,
(12) M9L / M202L / M323T / M382Y,
(13) M9L / M202L / Y295F / A339S,
(14) M9L / M202L / Y295F,
(15) M9L / M202L / A339S,
(16) M9L / M202L / Y295F / A339S,
(17) M9L / M202L / Y295F / A339S, E345R,
(18) M9L / G149A / M202L / Y295F / A339S / E345R,
(19) M9L / M202T,
(20) M9L / M202T / M323T,
(21) M9L / M202T / M323T / M382Y,
(22) M9L / M202T / Y295F / A339S,
(23) M9L / M202T / Y295F,
(24) M9L / M202T / A339S,
(25) M9L / M202T / Y295F / A339S,
(26) M9L / M202T / Y295F / A339S / E345R,
(27) M9L / G149A / M202T / Y295F / A339S / E345R,
(28) M9L / G149A / M202I / V214T / Y295F / N299Y / M323T / A339S / E345R,
(29) M9L / G149A / M202L / V214I / Y295F / M323T / A339S / E345R / M382Y,
(30) M9L / G149A / G182T / G186A / M202I / V214I / Y295F / N299Y / M323T / A339S,
(31) M9L / G149A / G182T / G186A / M202L / T257I / Y295F / N299Y / M323T / A339S / E345R,
(32) M9L / G149A / M202L / V214T / Y295F / N299Y / M323T / A339S / E345R,
(33) M9L / G149A / M202I / V214I / Y295F / M323T / A339S / E345R / M382Y,
(34) M9L / G149A / G182T / G186A / M202L / V214I / Y295F / N299Y / M323T / A339S,
(35) M9L / G149A / G182T / G186A / M202I / T257I / Y295F / N299Y / M323T / A339S / E345R
(36) M9L / G149A / M202I / V214T / Y295F / N299Y / M323T / A339S / E345R / N471 E,
(37) M9L / G149A / M202L / V214I / Y295F / M323T / A339S / E345R / M382Y / N471 E,
(38) M9L / G149A / G182T / G186A / M202I / V214I / Y295F / N299Y / M323T / A339S / N471 E,
(39) M9L / G149A / G182T / G186A / M202L / T257I / Y295F / N299Y / M323T / A339S / E345R / N471E,
(40) M202L / M105F / M208F,
(41) G133E / M202L / Q361E,
(42) G133E / M202L / R444E,
(43) M202L / Y295F,
(44) M202L / A339S,
(45) M202L / M323T,
(46) M202L / M323T / M309L,
(47) M202L / M323T / M430I,
(48) M202L / V214T / R444Y,
(49) M202L / N283D / Q361E,
(50) M202L / M382Y / K383R.
(51) M202L / K446R / N4840,
(52) M202I / Y295F,
(53) M202I / A339S,
(54) M202I / M105F / M208F,
(55) G133E / M202I / 0361E,
(56) G133E / M202I / R444E,
(57) M202I / M323T,
(58) M202I / M323T / M309L,
(59) M202I / M323T / M4301,
(60) M202I / V214T / R444Y,
(61) M202I / N283D / Q361E,
(62) M202I / M382Y / K383R,
(63) M202I / K446R / N484Q,
(64) M202V / M105F / M208F,
(65) G133E / M202V / 0361E,
(66) G133E / M202V / R444E,
(67) M202V / M323T,
(68) M202V / M323T / M309L,
(69) M202V / M323T / M430I,
(70) M202V / M323T / M9L,
(71) M202V / V214T / R444Y,
(72) M202V / N283D / 0361E,
(73) M202V / M382Y / K383R,
(74) M202V / K446R / N4840,
(75) M202T / M105F / M208F,
(76) G133E / M202T / 0361E,
(77) G133E / M202T / R444E,
(78) M202T / Y295F,
(79) M202T / A339S,
(80) M202T / M323T,
(81) M202T / M323T / M309L,
(82) M202T / M323T / M4301,
(83) M202T / M323T / M9L,
(84) M202T / V214T / R444Y,
(85) M202T / N283D / 0361E,
(86) M202T / A339S,
(87) M202T / Y295F
(88)) M202T / N299F,Y,
(89) M202T / M382Y / K383R ou
(90) M202T / K446R / N4840
